# EUROPÄISCHE PATENTSCHRIFT

(11) **EP 2 775 996 B1**
(45) Veröffentlichungstag und Bekanntmachung des Hinweises auf die Patenterteilung: **28.02.2018**
(21) Anmeldenummer: 12769327.3
(22) Anmeldetag: 27.09.2012
(51) Int. Cl.: A61K 9/00, A61K 8/34, A61K 31/065, A61K 31/085, A61P 17/10, A61P 17/18, A61Q 19/08, A61Q 5/00, A61Q 5/12, A61K 9/107, A61K 31/09

(54) **VERWENDUNG VON PROPANOL- UND PROPENOLDERIVATEN ALS ANTIOXIDANTIEN**
USE OF PROPANOL AND PROPENOL DERIVATIVES AS ANTIOXIDANTS
UTILISATION DE DÉRIVÉS DE PROPANOL ET DE PROPÉNOL EN TANT QU'ANTIOXYDANTS

(30) Priorität: 01.10.2011 DE 102011114780
(43) Veröffentlichungstag der Anmeldung: 17.09.2014
(73) Patentinhaber: Merck Patent GmbH, 64293 Darmstadt (DE)
(72) Erfinder: RUDOLPH, Thomas, 64291 Darmstadt (DE); SCHEURICH, René, Peter, 64846 Gross-Zimmern (DE)
(86) Internationale Anmeldenummer: PCT/EP2012/004061
(87) Internationale Veröffentlichungsnummer: WO 2013/045098

(56) Entgegenhaltungen:
- EP-A1- 1 701 938
- EP-A2- 2 227 233
- WO-A1-2004/087629
- JP-A- H05 213 729
- US-A1- 2010 267 839
- DATABASE REGISTRY CHEMICAL ABSTRACTS SERVICE, COLUMBUS, OHIO, US; 8. September 2011 (2011-09-08), XP002724101, Database accession no. RN 1330120-45-0
- DATABASE REGISTRY [Online] CHEMICAL ABSTRACTS SERVICE, COLUMBUS, OHIO, US; 7. September 2011 (2011-09-07), XP002725254, Database accession no. RN 1329637-29-7
- DATABASE REGISTRY [Online] CHEMICAL ABSTRACTS SERVICE, COLUMBUS, OHIO, US; 2. September 2011 (2011-09-02), XP002725255, Database accession no. RN 1327144-78-4, 1327144-74-0
- DATABASE REGISTRY [Online] CHEMICAL ABSTRACTS SERVICE, COLUMBUS, OHIO, US; 1. September 2011 (2011-09-01), XP002725256, Database accession no. RN 1326729-73-0, 1326729-20-7
- DATABASE REGISTRY [Online] CHEMICAL ABSTRACTS SERVICE, COLUMBUS, OHIO, US; 10. Juni 2011 (2011-06-10), XP002725257, Database accession no. RN 1308505-03-4
- DATABASE REGISTRY [Online] CHEMICAL ABSTRACTS SERVICE, COLUMBUS, OHIO, US; 7. Juni 2011 (2011-06-07), XP002725258, Database accession no. RN 1307156-19-9, 1307006-68-3
- DATABASE REGISTRY [Online] CHEMICAL ABSTRACTS SERVICE, COLUMBUS, OHIO, US; 11. Mai 2011 (2011-05-11), XP002725259, Database accession no. RN 1293001-01-0
- DATABASE REGISTRY [Online] CHEMICAL ABSTRACTS SERVICE, COLUMBUS, OHIO, US; 18. April 2001 (2001-04-18), XP002725260, Database accession no. RN 1281732-84-0
- DATABASE REGISTRY [Online] CHEMICAL ABSTRACTS SERVICE, COLUMBUS, OHIO, US; 6. April 2011 (2011-04-06), XP002725261, Database accession no. RN 1275863-68-7
- DATABASE REGISTRY [Online] CHEMICAL ABSTRACTS SERVICE, COLUMBUS, OHIO, US; 29. März 2011 (2011-03-29), XP002725262, Database accession no. RN 1271665-15-6
- DATABASE REGISTRY [Online] CHEMICAL ABSTRACTS SERVICE, COLUMBUS, OHIO, US; 4. Februar 2011 (2011-02-04), XP002725263, Database accession no. RN 1262130-14-2
- DATABASE REGISTRY [Online] CHEMICAL ABSTRACTS SERVICE, COLUMBUS, OHIO, US; 23. Dezember 2007 (2007-12-23), XP002725264, Database accession no. RN 959409-21-3
- SARVESH KUMAR ET AL: "Arylalkyl Ketones, Benzophenones, Desoxybenzoins and Chalcones Inhibit TNF-[alpha] Induced Expression of ICAM-1: Structure-Activity Analysis", ARCHIV DER PHARMAZIE, Bd. 345, Nr. 5, 20. Dezember 2011 (2011-12-20), Seiten 368-377, XP055116299, Weinheim ISSN: 0365-6233, DOI: 10.1002/ardp.201100279

## Beschreibung

Die Erfindung betrifft die Verwendung von Propanol- und Propenolderivaten als Antioxidantien, Zusammensetzungen enthaltend Propanol- und Propenolderivate und deren Herstellung, sowie bestimmte Propanol- und Propenolderivate und deren Herstellung.

Aufgabe pflegender Kosmetik ist es, nach Möglichkeit den Eindruck einer jugendlichen Haut zu erhalten. Prinzipiell stehen verschiedene Wege offen, um diesen Weg zu erreichen. So können bereits vorhandene Schädigungen der Haut, wie unregelmäßige Pigmentierung oder Faltenbildung, durch abdeckende Puder oder Cremes ausgeglichen werden. Ein anderer Ansatzpunkt ist, die Haut vor Umwelteinflüssen zu schützen, die zu einer dauerhaften Schädigung und damit Alterung der Haut führen.

So verliert beispielsweise mit zunehmendem Alter die Haut an Fähigkeit, Radikale, die durch äußere oder innere Stressfaktoren erzeugt werden, abzufangen. Diese sich abschwächenden Abwehrfunktion der Haut gegen schädigende Einflüsse kann zum Beispiel durch die äußere Zufuhr von Substanzen ausgeglichen werden, welche in der Lage sind, die nachlassenden Abwehr- bzw. Reparaturfunktionen zu ersetzen. Solche Substanzen sind beispielsweise Substanzen, die eine antioxidative Wirkung aufweisen und damit oxidativen Stress der Haut abbauen, bzw. diesem präventiv vorbeugen können.

Aufgabe der vorliegenden Erfindung war daher die Bereitstellung von Substanzen, die als Antioxidantien verwendet werden können, damit eine schützende Wirkung gegen oxidativen Stress aufweisen und den allgemeinen Zustand der Haut oder der Haare verbessern können.

Überraschend wurde nun gefunden, dass bestimmte Propanol- und Propenolderivate hervorragende Eigenschaften als Antioxidantien aufweisen.

Ausgewählte Propanol- und Propenolderivate sind in der Literatur bekannt. So wird zum Beispiel die Herstellung von unterschiedlich substituierten 1,3-Diphenyl-1-propanolderivaten in Kose et al, 2010, Org. Biomol. Chem. 8, 896-900 offenbart.

Die Synthese unterschiedlich substituierter 1,3-Diphenyl-1-propenolderivate wird exemplarisch beschrieben in Sakurai et al, 2009, Chem. Pharm. Bull. 57(5), 511-512.

Im Stand der Technik wird jedoch nicht die antioxidative Wirkung dieser Verbindungen beschrieben.

EP 1701938 A1 offenbart die Verwendung von 1,3-Diphenylpropanon-Derivaten zur Behandlung einer Reihe von Krankheiten, einschließlich der Behandlung von oxidativem Stress oder kosmetischen Alterungserscheinungen. Die Verbindungen weisen auch antioxidative Eigenschaften auf. Bei diesen Verbindungen handelt es sich um Ketoverbindungen.

Ein erster Gegenstand der vorliegenden Erfindung ist daher die nicht-therapeutische Verwendung einer oder mehrerer Verbindungen der Formel (1) und/ oder (2) 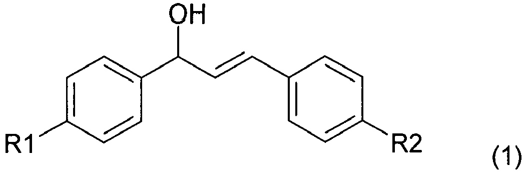 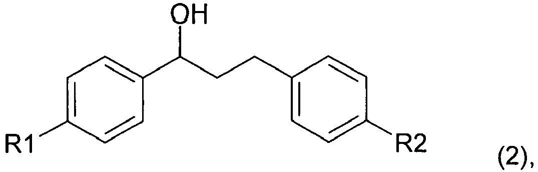 wobei R1 und R2 unabhängig voneinander für Wasserstoff, C1- bis C12-Alkoxy oder C1- bis C12-Alkyl stehen,
als Antioxidans.

Die dargestellte Strukturformel (1) umfasst erfindungsgemäß jeweils sowohl die trans- als auch die entsprechenden cis-lsomere, welche zum Beispiel photochemisch aus den trans-Isomeren gebildet werden können.

So können die Verbindungen der Formel (1) und/ oder (2) zur Prophylaxe gegen oder zur Bekämpfung von oxidativem Stress verwendet werden.

Der Abbau von oxidativem Stress resultiert in einer Reihe positiver Wirkungen: So können Verbindungen der Formel (1) und/ oder (2) auch zur Pflege, Konservierung oder Verbesserung des allgemeinen Zustands oder des Erscheinungsbildes der Haut oder Haare verwendet werden, oder zur Prophylaxe gegen oder zur Bekämpfung von zeit- und/oder lichtinduzierten Alterungsprozessen der Haut oder Haare.

Im Speziellen kann es sich dabei beispielsweise um die Prophylaxe gegen oder die Reduktion von trockener Haut, Faltenbildung und/oder Pigmentstörungen, und/oder schädigender Effekte von UV-Strahlen auf die Haut handeln, sowie um die Prophylaxe gegen oder die Reduktion von Hautunebenheiten, wie Falten, feinen Linien, rauher Haut oder großporiger Haut.

Die antioxidative Wirkung der Verbindungen ermöglicht auch die Prophylaxe gegen oder die Bekämpfung von Sebumoxidation und/oder Akne. Verbindungen der Formel (1) und/ oder (2) weisen damit auch eine anticomedogene bzw. anti-Akne-Wirkung auf.

Die Verbindungen können auch zur Stabilisierung anderer Formulierungsbestandteile wie UV-Filter, Farbstoffe und Vitamine verwendet werden.

Insbesondere sind durch die Verwendung der Verbindungen der Formeln (1) und/ oder (2) beispielsweise folgende Vorteile gegeben:
- antioxidante Wirkung gegen Radikale, die z.B. durch UV-Licht oder durch thermolytische Prozesse, wie dem Rauchen, induziert werden, wie z.B. gegen das Superoxidradikalanion oder das NO-Radikal, bzw. gegen reaktive Sauerstoffspezies, wie z. B. gegen Singulettsauerstoff und Peroxide;
- starke antioxidative Aktivität in Kombination mit einer hohen molekularen Stabilität gegen Umwelteinflüsse wie Wärme oder Licht, insbesondere UV-Licht, aber auch Infrarot und sichtbare Strahlung;
- produktstabilisierende Wirkung auf kosmetische, pharmazeutische, insbesondere dermatologische Produkte, insbesondere solche, die Färb-, Konsistenz-, Geruchsstoffe oder Vitamine enthalten;
- Verbindungen der Formeln (1) und (2) eignen sich zum DNA-Schutz. Sie können zudem die Zellproliferation und Zelldifferenzierung in der Haut fördern und so z. B. zur Anti-Ageing, Anti-Falten und wundheilenden Wirkung beitragen;
- Verbindungen der Formeln (1) und (2) eignen sich zur Erzeugung bzw. Erhöhung ("boost"-Effekt) von Lichtschutzfaktoren, wie LSF, SPF, PPD oder IPD, oder Radikalschutzfaktoren;
- stabilisierende Wirkung auf autooxidierbare Polyethylenglycol (PEG)-oder Polyglycerin (PG)-Derivate, wie insbesondere PEG- oder PG-haltige Emulgatoren, wie sie weiter unten in dieser Anmeldung genannt werden, bzw. eine Reduktion der schädigenden Wirkung der Abbauprodukte autooxidierbarer Polyethylenglycol (PEG)- oder Polyglycerin (PG)-Derivate;
- stabilisierende Wirkung auf Farb-, Konsistenz- oder Geruchsstoffe, oder auf Antioxidantien oder Vitamine, und UV Filter sowie Titandioxid-haltige Pigmente, insbesondere in kosmetische, pharmazeutische, insbesondere dermatologische Produkten;

Weitere Vorteile der Verbindungen der Formeln (1) und (2) sind ihre kostengünstige Zugänglichkeit und ihre vorteilhaften galenischen Eigenschaften: Die Verbindungen kristallisieren nicht aus, wenn sie in Zubereitungen eingearbeitet vorliegen, weisen keine Eigenfärbung auf und sind geruchsneutral.

Bevorzugt steht der Rest R1 in den Verbindungen der Formel (1) und (2) für Wasserstoff, C1- bis C6-Alkyl oder C1- bis C6-Alkoxy; besonders bevorzugt für Wasserstoff, C3- bis C5-Alkyl oder C1- bis C2-Alkoxy; ganz besonders bevorzugt für Wasserstoff, Methoxy, Isopropyl oder t-Butyl.

Bevorzugt steht der Rest R2 in den Verbindungen der Formel (1) und (2) für Wasserstoff, C1- bis C6-Alkyl oder C1- bis C6-Alkoxy; besonders bevorzugt für Wasserstoff, C3- bis C5-Alkyl oder C1-bis C2-Alkoxy; ganz besonders bevorzugt für Wasserstoff, Methoxy, Isopropyl oder t-Butyl.

Für die erfindungsgemäße Verwendung besonders bevorzugte Verbindungen der Formel (1) sind ausgewählt aus Verbindungen der Formel (1a) bis (1p), ganz besonders bevorzugt sind die Verbindungen (1a), (1b) und (1l), insbesondere (1a).

| | | | |
|---|---|---|---|
| 1a | 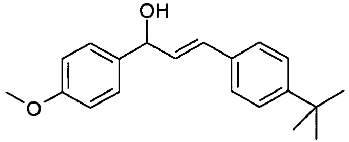 | 1b | 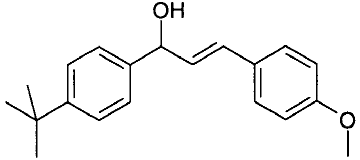 |
| 1c | 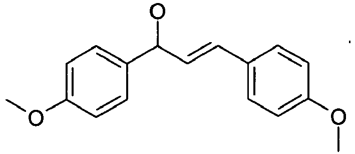 | 1d | 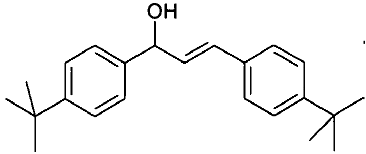 |
| 1e | 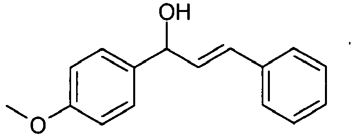 | 1f | 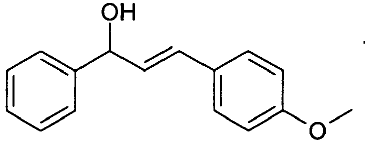 |
| 1g | 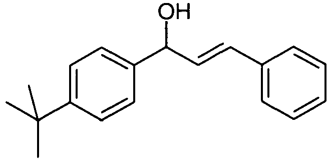 | 1h | 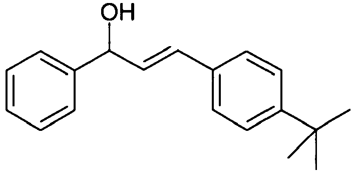 |
| 1i | 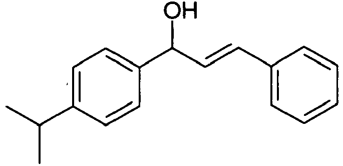 | 1j | 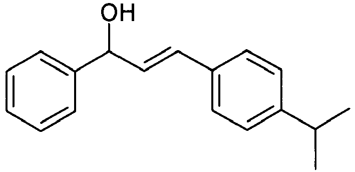 |
| 1k | 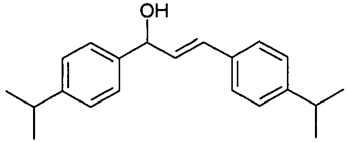 | 1l | 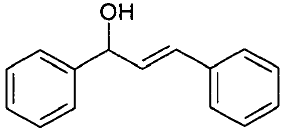 |
| 1m | 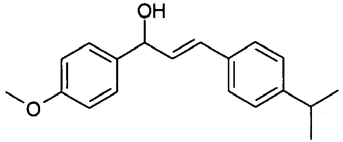 | 1n | 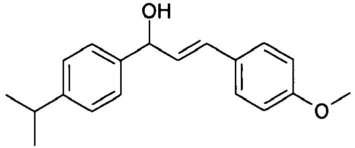 |
| 1o | 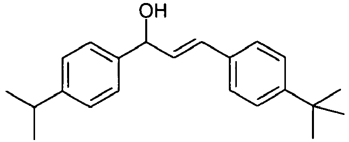 | 1p | 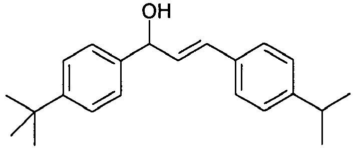 |

Für die erfindungsgemäße Verwendung besonders bevorzugte Verbindungen der Formel (2) sind ausgewählt aus Verbindungen der Formel (2a) bis (2p), ganz besonders bevorzugt sind die Verbindungen (2a), (2b) und (2l), insbesondere (2a).

| | | | |
|---|---|---|---|
| 2a | 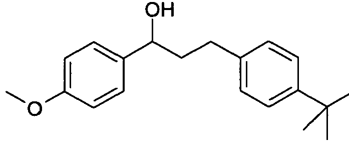 | 2b | 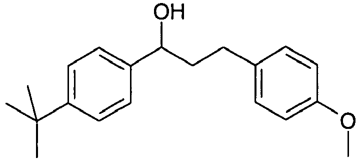 |
| 2c | 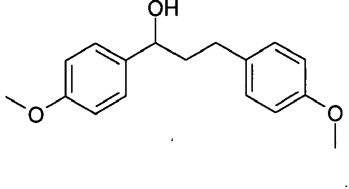 | 2d | 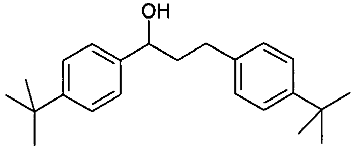 |
| 2e | 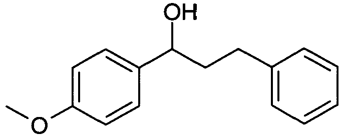 | 2f | 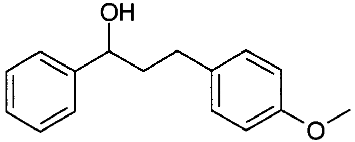 |
| 2g | 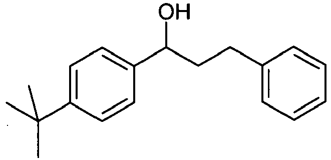 | 2h | 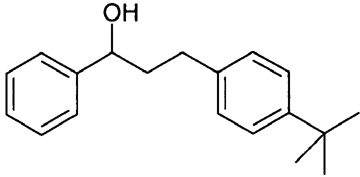 |
| 2i | 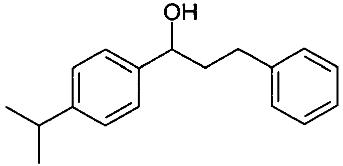 | 2j | 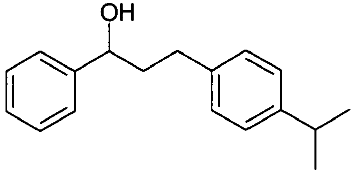 |
| 2k | 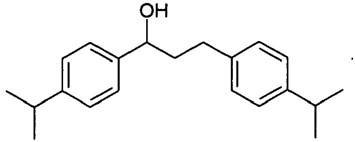 | 2l | 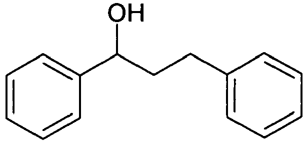 |
| 2m | 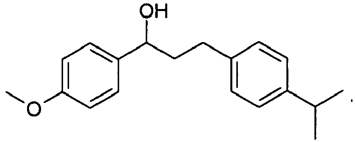 | 2n | 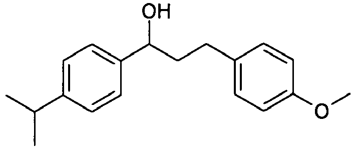 |
| 2o | 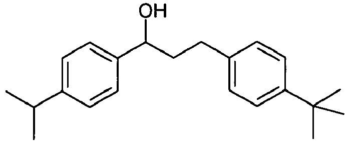 | 2p | 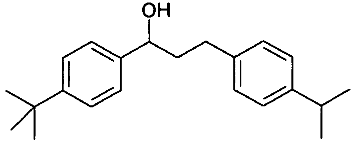 |

Die Verbindungen der Formel (1) und (2) können durch katalytische Hydrierung aus dem jeweils korrespondierenden Chalcon (1-A) bzw. Dihydrochalcon (2-A) der Formel 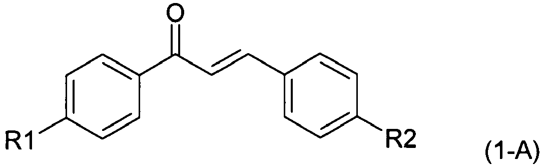 bzw. 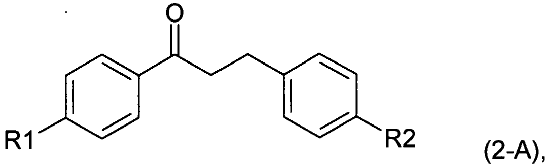 hergestellt werden,
wobei die Reste R1 und R2 wie zuvor für Formel (1) bzw. Formel (2) definiert sind.

Ferner lassen sich Verbindungen der Formel (2) auch durch katalytische Hydrierung aus dem Chalcon (1-A) herstellen.

Die möglichen Herstellungswege werden in den folgenden beiden Schemata veranschaulicht, wobei exemplarisch die Synthese der Verbindungen der Formel (1l) (Schema 1, erster Weg) bzw. (2l) (Schema 1, erster Weg; Schema 2) dargestellt wird. Die Reaktionsbedingungen sind den Schemata zu entnehmen.

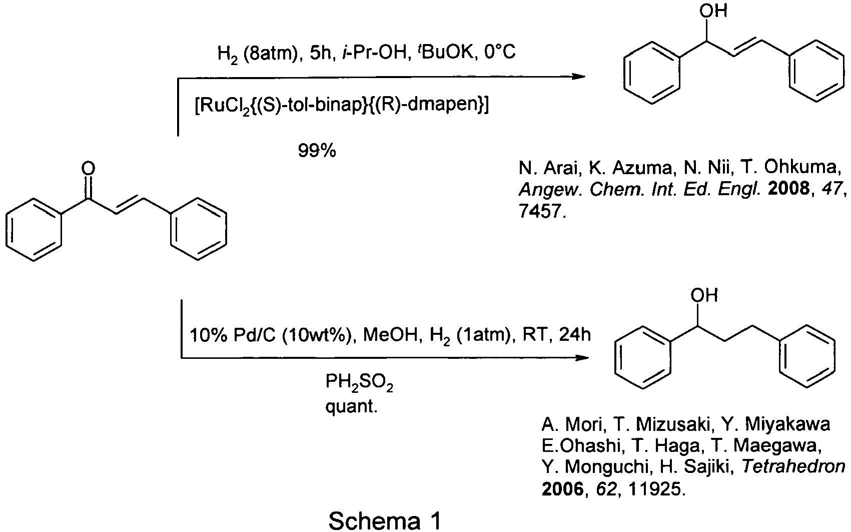

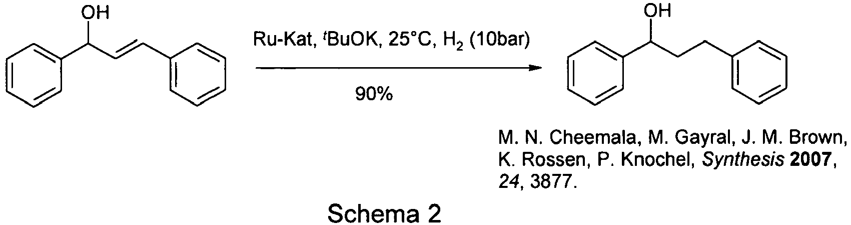

Die Ausgangsverbindungen der Formel (1-A) und (2-A) können beispielsweise auf dem in Schema 3 veranschaulichten Herstellungsweg erhalten werden:

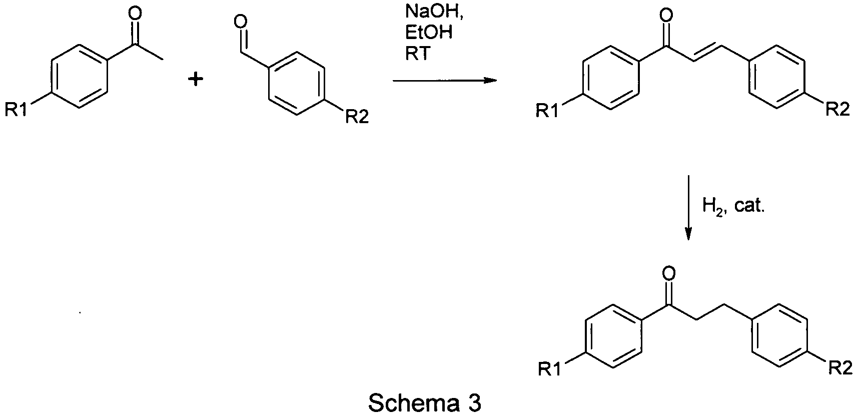

Alle weiteren Ausgangsmaterialien sind kommerziell erhältlich oder sind auf einfache und dem Fachmann bekannte Weise herstellbar.

Generell bereitet es dem Fachmann keinerlei Schwierigkeiten, die geeigneten Reaktionsbedingungen, wie Temperatur, Lösungsmittel oder Reaktionszeit auszuwählen.

Gemäß der vorliegenden Erfindung steht Alkyl als Abkürzung für Alkylgruppe. Die Alkylgruppe kann geradkettig oder verzweigt sein. Unter einem C1- bis C12-Alkyl wird erfindungsgemäß beispielsweise Methyl, Ethyl, Isopropyl, Propyl, Butyl, sek.-Butyl oder tert.-Butyl, Pentyl, 1-, 2- oder 3-Methylbutyl, 1,1-, 1,2- oder 2,2-Dimethylpropyl, 1-Ethylpropyl, Hexyl, Heptyl, 1-Ethyl-pentyl, Octyl, 1-Ethyl-hexyl, Nonyl, Decyl, Undecyl oder Dodecyl verstanden.

Unter einem C1- bis C6-Alkyl versteht man beispielsweise Methyl, Ethyl, Isopropyl, Propyl, Butyl, sek.-Butyl oder tert.-Butyl, Pentyl, 1-, 2- oder 3-Methylbutyl, 1,1-, 1,2- oder 2,2-Dimethylpropyl, 1-Ethylpropyl oder Hexyl. Ein C3- bis C5-Alkyl ist zum Beispiel Isopropyl, Propyl, Butyl, sek.-Butyl oder tert.-Butyl, Pentyl, 1-, 2- oder 3-Methylbutyl, 1,1-, 1,2- oder 2,2-Dimethylpropyl oder 1-Ethylpropyl.

Alkoxy steht als Abkürzung für Alkoxygruppe. Der Alkoxyrest kann linear oder verzweigt sein.

Beispiele für C1- bis C12-Alkoxygruppen sind ferner Methoxy, Ethoxy, Propoxy, Isopropoxy, Butoxy, Isobutoxy oder Tert.-Butoxy, 1,1-, 1,2- oder 2,2-Dimethylpropoxy, 1-Ethylpropoxy, Hexoxy, Heptoxy, 1-Ethyl-pentoxy, Octoxy, Nonoxy, Decoxy, Undecoxy, Dodecoxy, Tridecoxy, Tetradecoxy, Pentadecoxy, Hexadecoxy, Heptadecoxy, Octadecoxy oder Nonadecoxy. C1- bis C6-Alkoxy steht beispielsweise für Methoxy, Ethoxy, Propoxy, Isopropoxy, Butoxy, Isobutoxy oder Tert.-Butoxy, 1,1-, 1,2- oder 2,2-Dimethylpropoxy, 1-Ethylpropoxy oder Hexoxy.

Unter C1- bis C2-Alkoxy wird erfindungsgemäß Methoxy oder Ethoxy verstanden.

Gegenstand der vorliegenden Erfindung ist auch eine Zubereitung enthaltend mindestens eine Verbindung der Formel (1) und/ oder (2) 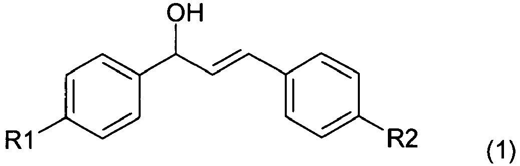 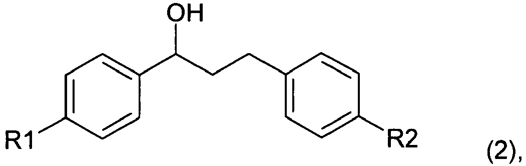 wobei R1 und R2 unabhängig voneinander für Wasserstoff, C1- bis C12-Alkoxy oder C1- bis C12-Alkyl stehen,
und mindestens einen für topische Anwendungen geeigneten Träger.

Bevorzugt stehen R1 und R2 für Reste wie zuvor definiert. Die Verbindungen der Formeln (1) und (2) sind dabei besonders bevorzugt ausgewählt aus den Verbindungen der Formel (1a) bis (1p) und (2a) bis (2p), wie oben definiert, ganz besonders bevorzugt aus den Verbindungen der Formel (1a), (1b), (1l), (2a), (2b) und (2l), insbesondere aus den Verbindungen der Formel (1a) und (2a).

Die Substanzen der vorliegenden Erfindung können dabei in den Formulierungen auch miteinander kombiniert werden.

Bei den Zubereitungen handelt es sich dabei üblicherweise um topisch anwendbare Zubereitungen, beispielsweise kosmetische oder dermatologische Formulierungen oder Medizinprodukte. Die Zubereitungen enthalten in diesem Fall einen kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitungen in diesem Fall einen pharmazeutisch verträglichen Träger und optional weitere pharmazeutische Wirkstoffe.

Im Sinne der vorliegenden Erfindung wird neben dem Begriff Zubereitung gleichbedeutend auch der Begriff Mittel oder Formulierung verwendet.

Die Zubereitungen können die genannten notwendigen oder optionalen Bestandteile umfassen oder enthalten, daraus im wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Bevorzugt handelt es sich um eine kosmetische oder pharmazeutische Zubereitung; besonders bevorzugt handelt es sich um eine kosmetische Zubereitung.

Gegenstand der vorliegenden Erfindung ist auch ein Verfahren zur Herstellung einer Zubereitung, wie zuvor beschrieben, dadurch gekennzeichnet, dass die mindestens eine Verbindung der Formel (1) und/ oder (2) mit dem für topische Anwendungen geeigneten Träger und gegebenenfalls mit weiteren Aktiv- oder Hilfsstoffen vermischt wird. Geeignete Trägerstoffe sowie Aktiv- oder Hilfsstoffe sind im nachfolgenden Teil ausführlich beschrieben.

In bevorzugten Ausführungsformen wird die mindestens eine Verbindung der Formel (1) und/oder (2) in den erfindungsgemäßen Zubereitungen typischerweise in Mengen von 0,01 bis 20 Gew.-%, bevorzugt in Mengen von 0,05 bis 10 Gew.-%, besonders bevorzugt in Mengen von 0,1 bis 5 Gew.-% und ganz besonders bevorzugt in Mengen von 0,5 bis 2 Gew.-%, eingesetzt. Dabei bereitet es dem Fachmann keinerlei Schwierigkeiten die Mengen abhängig von der beabsichtigten Wirkung der Zubereitung entsprechend auszuwählen.

Der Zusatz von Verbindungen, wie sie in WO 2007/121818 A1 offenbart werden, zu den erfindungsgemäßen Formulierungen ist besonders vorteilhaft, da dies eine gegenseitige Wirkverstärkung ermöglicht. Daher sind auch Zubereitungen eine mögliche Ausführungsform der vorliegenden Erfindung, die zusätzlich eine oder mehrere Verbindungen der Formel (3) 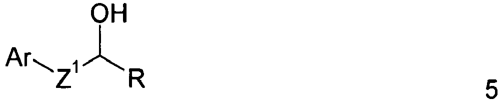 wobei R für 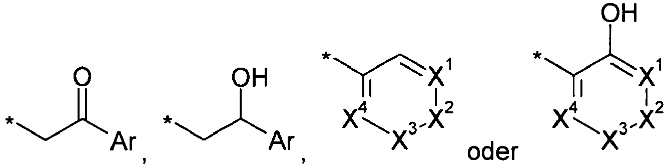 steht, wobei
Ar jeweils unabhängig voneinander stehen für einen unsubstituierten oder ein- oder mehrfach substituierten aromatischen Ring oder kondensierte Ringsysteme mit 6 bis 18C-Atomen, von denen mindestens ein Ring aromatischen Charakter besitzt, worin pro Ring auch ein oder zwei CH-Gruppen durch C=O, N, O oder S ersetzt sein können und in einem kondensierten Ringsystem auch ein oder zwei CH₂-Gruppen durch C=O oder C=CH₂ ersetzt sein können,
Z¹ steht für CR⁷R⁸ oder eine Einfachbindung,
X¹ bis X⁴ jeweils unabhängig voneinander ausgewählt sind aus C-R¹, O, N oder S, wobei 2 benachbarte Reste aus X¹ bis X⁴ gemeinsam auch stehen können für einen unsubstituierten oder ein- oder mehrfach substituierten Ring oder kondensierte Ringsysteme mit 6 bis 18C-Atomen, von denen vorzugsweise mindestens ein Ring aromatischen Charakter besitzt, worin pro Ring auch ein oder zwei CH-Gruppen durch C=O, N, O oder S ersetzt sein können und in einem kondensierten Ringsystem auch ein oder zwei CH₂-Gruppen durch C=O oder C=CH₂ ersetzt sein können,
R¹ ausgewählt ist aus H,
   geradkettigen oder verzweigten C₁-bis C₂₀-Alkoxygruppen, wobei die Alkylketten jeweils auch durch Sauerstoff oder Stickstoff unterbrochen sein können,
   geradkettigen oder verzweigten C₁-bis₂₀-Alkylgruppen, wobei die Alkylketten jeweils auch durch Sauerstoff oder Stickstoff unterbrochen sein können,
   geradkettigen oder verzweigten C₃-bis C₂₀-Alkenylgruppen, geradkettigen oder verzweigten C₁-bis C₂₀-Hydroxyalkylgruppen, wobei die Hydroxygruppe an ein primäres oder sekundäres Kohlenstoffatom der Kette gebunden sein kann und weiter die Alkylketten jeweils auch durch Sauerstoff oder Stickstoff unterbrochen sein können,
   geradkettigen oder verzweigten C₁-bis C₂₀-Hydroxyalkoxygruppen, wobei die Hydroxygruppe(n) an primäre oder sekundäre Kohlenstoffatome der Kette gebunden sein können und weiter die Alkylkette auch durch Sauerstoff unterbrochen sein kann, geradkettigen oder verzweigten C₁-bis C₂₀-Alkylaminogruppen, geradkettigen oder verzweigten C₁-bis C₂₀-Dialkylaminogruppen, oder R¹ steht für eine Carbonsäure-, Phosphorsäure-, Sulfonsäure-, Schwefelsäure- oder Sulfonfunktion, die optional mit geradkettigen oder verzweigten C₁-bis ₂₀-Alkylgruppen bzw. geradkettigen oder verzweigten C₃-bis C₂₀-Alkenylgruppen verestert oder alkyliert sein kann,
R⁷ und R⁸ jeweils unabhängig voneinander ausgewählt sind aus H, OH, geradkettigen oder verzweigten C₁-bisC₂₀-Alkoxygruppen, geradkettigen oder verzweigten C₁-bis ₂₀-Alkylgruppen, geradkettigen oder verzweigten C₃-bis C₂₀-Alkenylgruppen, geradkettigen oder verzweigten C₁-bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, geradkettigen oder verzweigten C₁-bis C₂₀-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann,
oder deren Salze enthalten.

Bevorzugt sind die Verbindungen der Formel (3) ausgewählt aus den Verbindungen der Formel (3a) und/oder (3b) 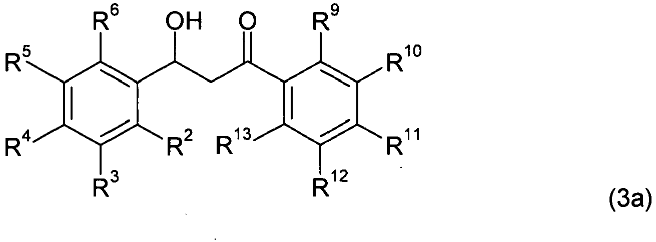 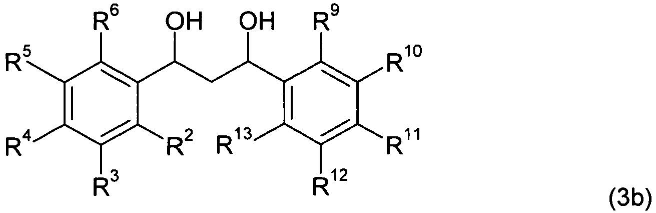 wobei
R⁴ und R¹¹ jeweils unabhängig voneinander H, geradkettige oder verzweigte C1- bis C20-Alkylgruppe, geradkettige oder verzweigte C1- bis C20-Alkoxygruppe oder geradkettige oder verzweigte C1- bis C20-Dialkylaminogruppe,
R⁶ H oder eine Carbonsäure-, Phosphorsäure-, Sulfonsäure-, Schwefelsäure- oder Sulfonfunktion, die mit geradkettigen oder verzweigten C1- bis C20-Alkylgruppen oder geradkettigen oder verzweigten C3- bis C20-Alkenylgruppen verestert oder alkyliert sein kann und
R², R³, R⁵, R⁹, R¹⁰, R¹² und R¹³ H bedeuten.

Besonders bevorzugt sind die Verbindungen der Formel (3a) und (3b) ausgewählt aus den Verbindungen umfassend 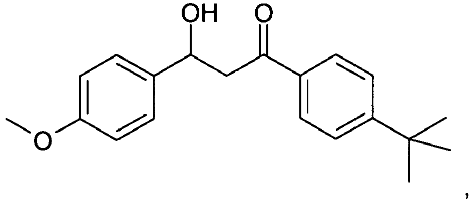 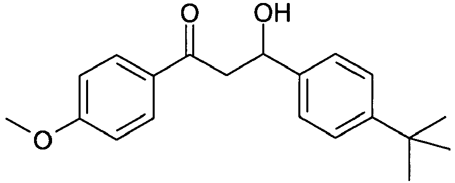 und

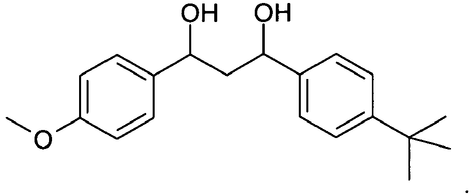

In den beschriebenen Zubereitungen, die erfindungsgemäß mindestens eine Verbindung der Formel (1) und/ oder (2) enthalten, können weiterhin auch Farbpigmente enthalten sein, wobei der Schichtaufbau der Pigmente nicht limitiert ist.

Vorzugsweise sollte das Farbpigment bei Einsatz von 0,5 bis 5 Gew.-% hautfarben oder bräunlich sein. Die Auswahl eines entsprechenden Pigments ist für den Fachmann geläufig.

Bevorzugte Zubereitungen enthalten neben der mindestens einen Verbindung der Formel (1) und/ oder (2) sowie den gegebenenfalls anderen Inhaltsstoffen UV-Filter.

Organische UV-Filter, sogenannte hydrophile oder lipophile Sonnenschutzfilter, sind im UVA-Bereich und/oder UVB-Bereich und/oder IR und/oder VIS-Bereich (Absorber) wirksam. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben. Im Folgenden werden die genannten UV-Filter meist nach der INCI-Nomenklatur benannt.

Insbesondere für eine Kombination geeignet sind:
para-Aminobenzoesäure und deren Derivate: PABA, Ethyl PABA, Ethyl dihydroxypropyl PABA, Ethylhexyl dimethyl PABA, z. B. vetrieben unter dem Namen "Escalol 507" von der Fa. ISP, Glyceryl PABA, PEG-25 PABA, z. B. vetrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vetrieben unter dem Namen "Neo Heliopan OS" von der Fa. Symrise, Dipropylene glycol salicylate, z. B. vetrieben unter dem Namen "Dipsal" von der Fa. Scher, TEA salicylate, z. B. vetrieben unter dem Namen "Neo Heliopan TS" von der Fa. Symrise.

β,β-Diphenylacrylate Derivate: Octocrylene, z. B. vetrieben unter dem Namen "Eusolex® OCR" von der Firma Merck", "Uvinul N539" von der Fa. BASF, Etocrylene, z. B. vetrieben unter dem Namen "Uvinul N35" von der BASF.

Benzophenone Derivate: Benzophenone-1, z. B. vetrieben unter dem Namen "Uvinul 400"; Benzophenone-2, z. B. vetrieben unter dem Namen "Uvinul D50" ; Benzophenone-3 oder Oxybenzone, z. B. vetrieben unter dem Namen "Uvinul M40";Benzophenone-4, z. B. vetrieben unter dem Namen "Uvinul MS40" ; Benzophenone-9, z. B. vetrieben unter dem Namen "Uvinul DS-49" von der Fa. BASF, Benzophenone-5, Benzophenone-6, z. B. vetrieben unter dem Namen "Helisorb 11" von der Fa. Norquay, Benzophenone-8, z. B. vetrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.

Benzylidencampher Derivate: 3-Benzylidenecamphor, z. B. vetrieben unter dem Namen "Mexoryl SD" von der Fa. Chimex, 4-Methylbenzylidenecamphor, z. B. vetrieben unter dem Namen "Eusolex 6300" von der Fa. Merck, Benzylidenecamphorsulfonsäure, z. B. vetrieben unter dem Namen "Mexoryl SL" von der Fa. Chimex, Camphor benzalkonium methosulfate, z. B. vetrieben unter dem Namen "Mexoryl SO" von der Fa. Chimex, Terephthalylidenedicamphorsulfonsäure, z. B. vetrieben unter dem Namen "Mexoryl SX" von der Fa Chimex, Polyacrylamidomethylbenzylidenecamphor vetrieben unter dem Namen "Mexoryl SW" von der Fa. Chimex.

Phenylbenzimidazol Derivate: Phenylbenzimidazolesulfonsäure, z. B. vetrieben unter dem Namen "Eusolex 232" von der Fa. Merck, Dinatrium phenyl dibenzimidazole tetrasulfonat, z. B. vetrieben unter dem Namen "Neo Heliopan AP" von der Fa. Symrise.

Phenylbenzotriazol Derivate: Drometrizole trisiloxane, z. B. vetrieben unter dem Namen "Silatrizole" von der Fa. Rhodia Chimie, Methylenebis(benzotriazolyl)tetramethylbutylphenol in fester Form, z. B. vetrieben unter dem Namen "MIXXIM BB/100" von der Fa. Fairmount Chemical, oder in mikronisierter Form als wässrige Dispersion, z. B. vetrieben unter dem Namen "Tinosorb M" von der Fa. BASF.

Triazin Derivate: Ethylhexyltriazone, z. B. vetrieben unter dem Namen "Uvinul T150" von der Fa. BASF, Diethylhexylbutamidotriazone, z. B. vetrieben unter dem Namen "Uvasorb HEB" von der Fa. Sigma 3V, 2,4,6-tris(diisobutyl 4'-aminobenzalmalonate)-s-triazine oder 2,4,6-Tris-(biphenyl)-1,3,5-triazine vertrieben als Tinosorb A2B von BASF, 2,2'-[6-(4-methoxyphenyl)-1,3,5-triazine-2,4-diyl]bis[5-(2-ethylhexyl)oxy]-phenol, vertrieben als Tinosorb S von BASF, N2,N4-bis[4-[5-(1,1-dimethylpropyl)-2-benzoxazolyl]phenyl]-N6-(2-ethylhexyl)-1,3,5-triazin-2,4,6-triamin vertrieben als Uvasorb K 2A von der Firma Sigma 3V.

Anthranilin Derivate: Menthyl anthranilate, z. B. vetrieben unter dem Namen "Neo Heliopan MA" von der Fa. Symrise.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vetrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vetrieben unter dem Namen Uvasorb K2A von der Fa. Sigma 3V und Mischungen dieses enthaltend.

Piperazinderivate wie beispielsweise die Verbindung 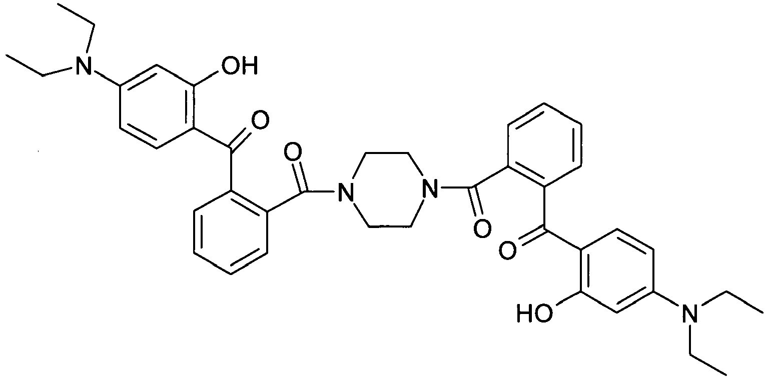 oder die UV-Filter der folgenden Strukturen 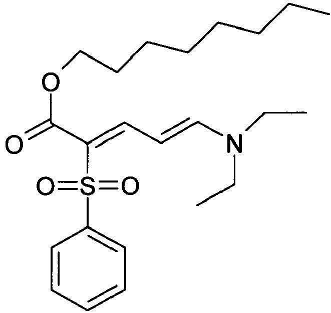 oder

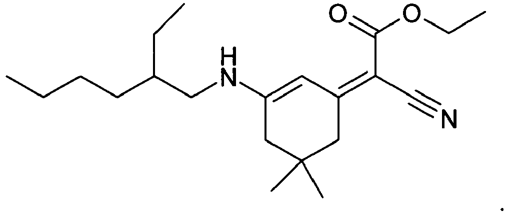

Es können auch UV-Filter auf Basis von Polysiloxancopolymeren mit einer statistischen Verteilung gemäß nachfolgender Formel verwendet werden, wobei z.B. a = 1,2; b= 58 und c=2,8 sind:

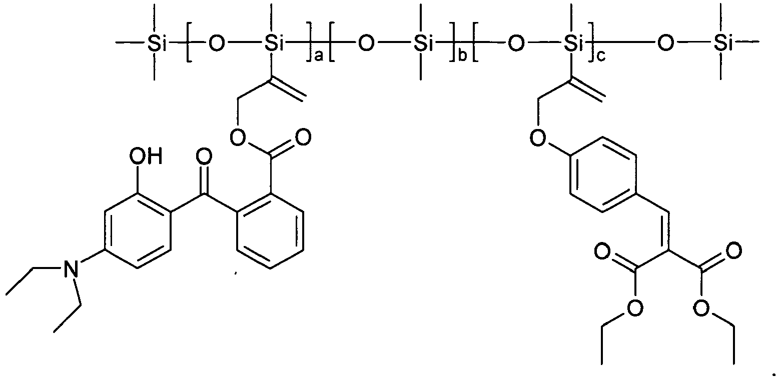

Die in der Liste aufgeführten Verbindungen sind nur als Beispiele aufzufassen. Selbstverständlich können auch andere UV-Filter verwendet werden.

Geeignete organischen UV-schützende Substanzen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Bevorzugte Zubereitungen enthalten neben den Verbindungen der Formel (1) und/oder (2) sowie den gegebenenfalls organischen UV-Filtern, wie zuvor beschrieben, weitere anorganische UV-Filter, sogenannte partikuläre UV-Filter, enthalten.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex® T-2000, Eusolex®T-AQUA, Eusolex®T-AVO, Eusolex®T-OLEO), Zinkoxide (z.B. Sachtotec®), Eisenoxide oder auch Ceroxide und/oder Zirkonoxide bevorzugt.

Ferner sind auch Kombinationen mit pigmentärem Titandixoxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53 64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt eingesetzte partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexameta¬phosphate, wie z.B. das Produkt "Microtitanium Dioxide MT 150 W" der Fa. Tayca.

Die zur Kombination eingesetzten behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   - "Zinc Oxide CS-5" der Fa. Toshibi (ZnO nachbehandelt mit polymethylhydrogenosiloxane)
   - Nanogard Zinc Oxide FN der Fa. Nanophase Technologies
   - "SPD-Z1" der Fa Shin-Etsu (ZnO nachbehandelt mit einem Silikongepfropften Acrylpolymer, dispergiert in Cyclodimethylsiloxane
   - "Escalol Z100" der Fa ISP (Aluminiumoxid nachbehandeltes ZnO dispergiert in einer ethylhexyl methoxycinnamate/PVP-hexadecene/ methicone copolymer Mischung)
   - "Fuji ZNO-SMS-10" der Fa. Fuji Pigment (ZnO nachbehandelt mit Siliciumdioxid und Polymethylsilesquioxan);
   - Unbehandeltes Ceroxide Mikropigment z.B. mit der Bezeichnung "Colloidal Cerium Oxide" der Fa Rhone Poulenc
   - Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandeltem Titandioxid, Zinkoxid-Mischungen wie z.B . das Produkt UV-Titan M261 der Fa. Sachtleben verwendet werden.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen.

Dabei sind die Kapseln in erfindungsgemäß einzusetzenden Zubereitungen vorzugsweise in solchen Mengen enthalten, die gewährleisten, dass die verkapselten UV-Filter in den oben angegebenen Gewichtsprozentverhältnissen in der Zubereitung vorliegen.

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

Daher sind Zubereitungen bevorzugt, in denen mindestens ein weiteres Antioxidans enthalten ist.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA, Pentasodium ethylenediamin tetramethylen phosphonat und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selen¬methionin), Stilbene und deren Derivate (z.B. Stilbenoxid, trans-Stilbenoxid).

Geeignete Antioxidantien sind auch Verbindungen der Formeln A oder B 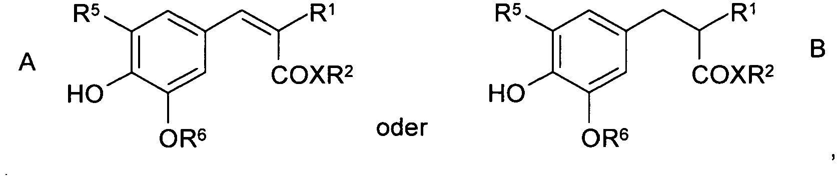 worin
- R¹: aus der Gruppe -C(O)CH₃, -CO₂R³, -C(O)NH₂ und -C(O)N(R⁴)₂ ausgewählt werden kann,
- X: O oder NH,
- R²: lineares oder verzweigtes Alkyl mit 1 bis 30 C-Atomen,
- R³: lineares oder verzweigtes Alkyl mit 1 bis 20 C-Atomen,
- R⁴: jeweils unabhängig voneinander H oder lineares oder verzweigtes Alkyl mit 1 bis 8 C-Atomen,
- R⁵: H, lineares oder verzweigtes Alkyl mit 1 bis 8 C-Atomen oder lineares oder verzweigtes Alkoxy mit 1 bis 8 C-Atomen und
- R⁶: lineares oder verzweigtes Alkyl mit 1 bis 8 C-Atomen bedeutet,
vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex® ST Liquid) und/oder 2-(4-Hydroxy-3,5-dimethoxybenzyl)-malonsäure-bis-(2-ethylhexyl)ester (z.B. RonaCare® AP).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L (+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit den erfindungsgemäßen Zusammensetzungen üblicherweise in in Mengen von 0,1 bis 20 Gew.-%, bevorzugt in Mengen von 0,1 bis 10 Gew.-% eingesetzt.

Unter den Phenolen, die erfindungsgemäß verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydroxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH Gruppen in 3'4'-oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers und I.M.C.M. Rietjens (Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Bevorzugte Zubereitungen können ebenfalls mindestens einen weiteren kosmetischen Wirkstoff enthalten, beispielsweise ausgewählt aus Anti-aging-, Anti-Falten-, Anti-Schuppen-, Anti-Akne-Wirkstoffen, antimikrobiellen Wirkstoffen, anti-inflammatorischen Wirkstoffen, Anti-Cellulite Wirkstoffen, Selbstbräunungssubstanzen, hautaufhellenden Wirkstoffen, Wirkstoffen zur Verbesserung des Feuchtegehaltes der Haut (Hautfeuchteregulatoren) oder Vitaminen.

Bei den Selbstbräunungssubstanzen kann es sich sowohl um einen Selbstbräuner handeln, der mit den Aminosäuren der Haut im Sinne einer Maillard-Reaktion oder über eine Michael-Addition reagiert, als auch um einen sogenannten Melanogenese-Promotor oder Propigmentierungswirkstoff, der die natürliche Bräunung der Haut fördert. Als vorteilhafte Selbstbräunungssubstanzen können unter anderem eingesetzt werden:
1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist das 1,3-Dihydroxyaceton, Erythrulose oder deren Kombination.

Propigmentierungsstoffe können prinzipiell alle dem Fachmann bekannten Wirkstoffe sein. Beispiele hierfür sind Glycyrrhetinsäure, Melanocytenstimulierendes Hormon (alpha-MSH), Peptidanaloga, Thymidin-Dinucleotide, L-Tyrosin und dessen Ester oder bicyclische Monoterpendiole (beschrieben in Brown et al., Photochemistry and Photobiology B: Biology 63 (2001) 148-161).

Bevorzugt ist die mindestens eine Selbstbräunungssubstanz in der Zubereitung in einer Menge von 0,01 bis 20 Gew.-%, besonders bevorzugt in einer Menge von 0,5 bis 15 Gew.-% und ganz besonders bevorzugt in einer Menge von 1 bis 8 Gew.-%, bezogen auf die Gesamtmenge der Zubereitung, enthalten.

Zubereitungen mit Selbstbräunereigenschaften, insbesondere solche, die Dihydroxyaceton enthalten, neigen bei der Anwendung auf der menschlichen Haut zu Fehlgerüchen, die vermutlich durch Abbauprodukte des Dihydroxyacetons selbst oder durch Produkte von Nebenreaktionen verursacht werden und die von den Anwendern teilweise als unangenehm empfunden werden. Es hat sich gezeigt, dass diese Fehlgerüche bei Verwendung von Formaldehydfängern und/oder Flavonoiden vermieden werden. Daher kann die erfindungsgemäße Zubereitung auch Formaldehydfänger sowie gegebenenfalls Flavonoide zur Verbesserung des Geruches enthalten.

Geeignete anti-aging Wirkstoffe, insbesondere für hautpflegende Zubereitungen, sind vorzugsweise sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1-Diglycerin-Phosphat (DGP), ß-Mannosylglycerat (Firoin), ß-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Additional können als anti-aging Wirkstoffe Produkte der Firma Merck wie z.B. 5,7-Dihydroxy-2-methyl-chromon, vermarktet unter dem Handelsnamen RonaCare®Luremine, Ronacare®Isoquercetin, Ronacare®Tilirosid oder Ronacare®Cyclopeptide 5 verwendet werden.

Die Zubereitungen können auch ein oder mehrere hautaufhellende Wirkstoffe oder synonym Depigmentierungswirkstoffe enthalten. Hautaufhellende Wirkstoffe können prinzipiell alle dem Fachmann bekannte Wirkstoffe sein. Zur Kombination eignen sich marktübliche Melanogeneseinhibitoren wie z.B. Ascorbinsäure und deren Derivate, Aloesin, Niacinamid, Emblica, Elaginsäure, Licorice-Extrakt, Maulbeerbaumextrakt, Kojisäure, Süßholzwurzelextrakt, Rucinol, Hydrochinon und dessen Derivate, Azelainsäure, Arbutin, Magnesiumascorbyl-phosphat, Pantothensäure und deren Derivate oder Salze, Glucosamin und dessen Derivate, Mercaptoamine, Hinokitol, Tocopherole, Ubichinone, Glabridin, 4-Hydroxyanisol, 4-tert-Butylphenol, Resveratrol, 4-S-Cystaminylphenol, Glutathion, Milchsäure oder dergleichen. Bevorzugte Beispiele von Verbindungen mit hautaufhellender Aktivität sind Hydrochinon, Niacinamid, Ascorbinsäure und physiologisch unbedenkliche Salze davon, Kojisäure, Arbutin, Aloesin, Azelainsäure, Elaginsäure oder Rucinol. Bevorzugte Beispiele von Extrakten mit hautaufhellender Aktivität sind Licorice-Extrakt, Maulbeerbaumextrakt oder Emblica.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt.

Die beschriebenen Retinoide sind gleichzeitig auch wirksame Anti-Cellulite-Wirkstoffe. Ein ebenfalls bekannter Anti-Cellulite-Wirkstoff ist Koffein.

Substanzen, die der Aufrechterhaltung und/oder der Verbesserung des Feuchtigkeitsgehaltes der Haut dienen, können, ohne dass dies als Einschränkung aufgefasst werden soll, unter anderem auch Substanzen sein, die zu den sogenannten natürlichen Feuchtigkeitsfaktoren (natural moisturizing factors) gehören, wie z.B. 2-Oxopyrrolidine 5-carbonsäure.

Die genannten Bestandteile der Zubereitung können in der üblichen Weise eingearbeitet werden, mit Hilfe von Techniken, die dem Fachmann wohl bekannt sind.

Geeignet sind Zubereitungen für eine äußerliche Anwendung, beispielsweise als Creme oder Milch (O/W, W/O, O/W/O, W/O/W), als Lotion oder Emulsion, in Form ölig-alkoholischer, ölig-wässriger oder wässrig-alkoholischer Gele bzw. Lösungen auf die Haut aufgesprüht werden kann. Sie können als feste Stifte vorliegen oder als Aerosol konfektioniert sein.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays.

Bevorzugte Hilfsstoffe stammen aus der Gruppe der Konservierungsstoffe, Stabilisatoren, Lösungsvermittler, Färbemittel, Geruchsverbesserer.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3 Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methyl-cyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C Zahl, z.B. mit Isopropanol, Propylenglykol oder Glycerin, oder Ester von Fettalkoholen mit Alkansäuren niedriger C Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C Atomen und gesättigten und/oder ungesättigten, verzweigten und/oder unverzweigten Alkoholen einer Kettenlänge von 3 bis 30 C Atomen, aus der Gruppe der Ester aus aromatischen Carbonsäure und gesättigten und/oder ungesättigten, verzweigten und/oder unverzweigten Alkoholen einer Kettenlänge von 3 bis 30 C Atomen. Solche Esteröle können dann vorteilhaft gewählt werden aus der Gruppe Isopropylmyristat, Isopropylpalmitat, Isopropylstearat, Isopropyloleat, n Butylstearat, n-Hexyllaurat, n Decyloleat, Isooctylstearat, Isononylstearat, Isononylisononanoat, 2 Ethylhexylpalmitat, 2 Ethylhexyllaurat, 2 Hexaldecylstearat, 2 Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C Zahl, sowie deren Ether, vorzugsweise Ethanol, Isopropanol, Propylenglykol, Glycerin, Ethylenglykol, Ethylenglykolmonoethyl oder -monobutylether, Propylenglykolmonomethyl, -monoethyl oder -monobutylether, Diethylenglykolmonomethyl oder-monoethylether und analoge Produkte, ferner Alkohole niedriger C Zahl, z. B. Ethanol, Isopropanol, 1,2 Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasser-in-ÖI (W/O) oder vom Typ Öl-in-Wasser (O/W), eine multiple Emulsion, beispielsweise vom Typ Waser-in-ÖI-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE A-43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen.

Es ist ferner von Vorteil, die Fettsäureethoxylate aus folgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat,
Polyethylenglycol(21)glyceryllaurat, Polyethylenglycol(22)glyceryllaurat,
Polyethylenglycol(23)glyceryllaurat,
Polyethylenglycol(6)glycerylcaprat/cprinat,
Polyethylenglycol(20)glyceryloleat, Polyethylenglycol(20)glycerylisostearat,
Polyethylenglycol(18)glyceryloleat(cocoat) zu wählen.

Es ist ebenfalls günstig, die Sorbitanester aus der Gruppe
Polyethylenglycol(20)sorbitanmonolaurat,
Polyethylenglycol(20)sorbitanmonostearat,
Polyethylenglycol(20)sorbitanmonoisostearat,
Polyethylenglycol(20)sorbitanmonopalmitat,
Polyethylenglycol(20)sorbitanmonooleat zu wählen.

Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C Atome, Diglycerinester gesättigter und/oder ungesättigter, verzweigter und/oder unverzweigter Alkancarbonsäuren einer Kettenlänge von 8 bis 24, insbesondere 12-18 C¬ Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B.

Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane , bevorzugt Alkane.

Ein weiterer Gegenstand der vorliegenden Erfindung sind auch Verbindungen der Formel (1) oder (2) 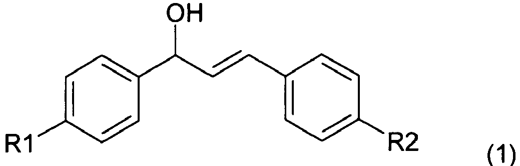 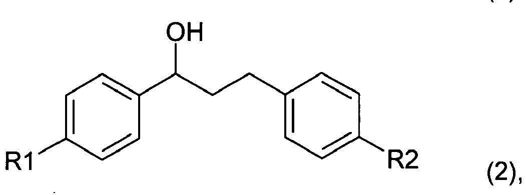 wobei R1 und R2 unabhängig voneinander für Wasserstoff, C1- bis C12-Alkoxy oder C1- bis C12-Alkyl stehen,
wobei Verbindungen der Formel (1) ausgeschlossen sind, in denen
R1=R2=H (1l),
R1=R2=Methyl,
R1=R2=Ethyl,
R1=R2=i-Butyl,
R1=R2=Methoxy (1c),
R1=Methyl, R2=H,
R1=H, R2=Methyl,
R1=Ethyl, R2=H,
R1=n-Heptyl, R2=H,
R1=Methoxy, R2=H (1e),
R1=H, R2=Methoxy (1f),
R1=Methyl, R2=Methoxy oder
R1=Methoxy, R2=Methyl,
und wobei Verbindungen der Formel (2) ausgewählt sind aus den Verbindungen 2a, 2b, 2d, 2k, 2m, 2o und 2p.

Bevorzugte Ausführungsformen der Reste R1 und R2 sind wie zuvor beschrieben definiert. Insbesondere sind Verbindungen der Formel (1) bevorzugt, ausgewählt aus den Verbindungen der Formel (1a), (1b), (1d), (1g), (1h), (1i), (1j), (1k), (1m), (1n), (1o) und (1p), wobei die genannten Verbindungen wie zuvor definiert sind. Ganz besonders bevorzugt sind die Verbindungen (1a), (1b), (2a) und (2b), insbesondere (1a) und (2a).

Ein weiterer Gegenstand der vorliegenden Erfindung ist auch ein Verfahren zur Herstellung einer Verbindung der Formel (1) oder (2), wie zuvor definiert, dadurch gekennzeichnet, dass eine Verbindung der Formel (1-A) oder (2-A) 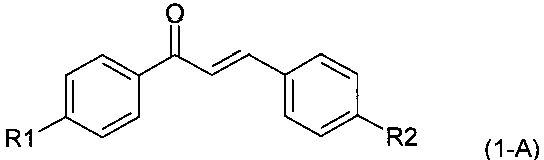 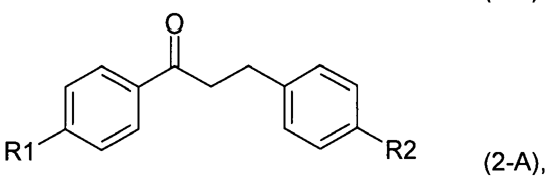 wobei die Reste R1 und R2 wie für Formel (1) bzw. Formel (2) definiert sind,
durch katalytische Hydrierung zu einer Verbindung der Formel (1) oder (2) umgesetzt wird.

Dabei wird eine Verbindung der Formel (2-A) in eine Verbindung der Formel (2) umgesetzt. Eine Verbindung der Formel (1-A) kann durch katalytische Hydrierung sowohl in eine Verbindung der Formel (1), als auch in eine Verbindung der Formel (2) umgesetzt werden.

Bezüglich Details betreffend das Herstellungsverfahren wird auf die Ausführungen weiter oben verwiesen.

Auch ohne weitere Ausführungen wird davon ausgegangen, dass ein Fachmann die obige Beschreibung in weitestem Umfang nutzen kann. Die bevorzugten Ausführungsformen und Beispiele sind deswegen lediglich als beschreibende, keinesfalls als in irgendeiner Weise limitierende Offenbarung aufzufassen.

Die Gewichtsprozentverhältnisse der einzelnen Inhaltsstoffe in den Zubereitungen der Beispiele gehören ausdrücklich zur Offenbarung der Beschreibung und können daher als Merkmale herangezogen werden.

Die im Folgenden angeführten Beispiele für den erfindungsgemäßen Gegenstand dienen lediglich der Erläuterung und engen die vorliegende Erfindung keineswegs in irgendeiner Weise ein. Im Übrigen ist die beschriebene Erfindung im gesamten beanspruchten Bereich ausführbar. Alle Verbindungen oder Komponenten, die in den Zubereitungen verwendet werden können, sind entweder bekannt und käuflich erhältlich oder können nach bekannten Methoden synthetisiert werden. Es werden in der Regel die INCI-Namen der verwendeten Rohstoffe angegeben (die INCI-Namen werden definitionsgemäß in englischer Sprache angegeben).

### Beispiele:

### Beispiel 1:

### Synthese von 3-(4-tert-Butyl phenyl)-1-(4-methoxy-phenyl)-prop-2-en-1-ol (Substanz 1a)

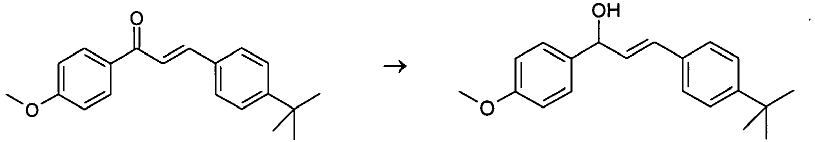

1mmol E-3-(4-tert-Butyl-phenyl)-1-(4-methoxy-phenyl)-propenon wird in einem Gemisch aus 15,0 mL 2-Propanol und 5,0 mL N,N-Dimethylformamid gelöst. Anschließend werden 10,0 mg Ruthenium(II) Katalysator und 6,0 mg Kalium-tert-butylat zugegeben. Die Hydrierung erfolgt mit Wasserstoff 3.0 bei 0°C und 8bar Druck. Nach vollständiger Hydrierung wird der Katalysator durch Filtration abgetrennt. Das Filtrat wird im Vakuum vom Lösungsmittel befreit und der Rückstand gewaschen. Die organische Phase wird über Natriumsulfat getrocknet und das Lösungsmittel im Vakuum entfernt. Die Reinigung erfolgt durch Filtration über Kieselgel. Man erhält analysenreines Produkt.

### Beispiel 2:

### Synthese von 3-(4-tert-Butyl-phenyl)-1-(4-methoxy-phenyl)-propan-1-ol (Substanz 2a)

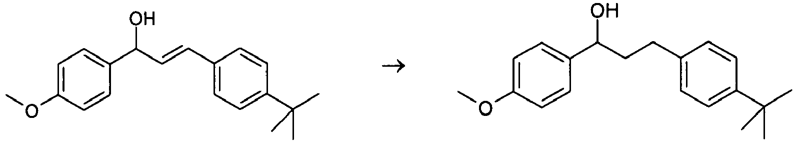

Zu 1mmol E-3-(4-tert-Butyl phenyl)-1-(4-methoxy-phenyl)-prop-2-en-1-ol werden 20mL Tetrahydrofuran (THF) gegeben sowie gelöst. Nach Zugabe des Katalysators Pd-C-5% (Pd-C-5% (56% Wasser; Merck: Art.-No. 275175; 0,54 g/mmol)) erfolgt die Hydrierung mit Wasserstoff 3.0 bei Raumtemperatur und Normaldruck. Der Katalysator wird durch Filtration abgetrennt. Das Filtrat wird im Vakuum vom Lösungsmittel befreit und der Rückstand gewaschen. Die organische Phase wird über Natriumsulfat getrocknet und das Lösungsmittel im Vakuum entfernt. Die Reinigung erfolgt durch Filtration über Kieselgel. Man erhält analysenreines Produkt.

### Beispiel 3:

Nachweis der reduzierenden (= antioxidativen) Eigenschaft von E-1,3-Diphenyl-2-propen-1-ol (Substanz 1l): 14mg von E-1,3-Diphenyl-2-propen-1-ol (Substanz 1l) werden in 10g MTB-Ether gelöst. Sowohl die Substanzlösung als auch das reine Lösungsmittel werden auf eine DC-Platte aufgetragen, die zuvor mit Kaliumpermanganatlösung besprüht wurde (DC Platte TLC Silica gel 60F254; 5g Kaliumpermanganat und 20g NaOH in 500 mL dest. Wasser). 30 Sekungen nach dem Substanzauftrag entfärbt sich die Kaliumpermanganatlösung im Bereich des Substanzauftrages, während das zum Vergleich aufgetragene Lösungmittel die violette Permanganatfärbung nicht entfärbt.

### Beispiel 4:

Nachweis der reduzierenden (= antioxidativen) Eigenschaft von 3-(4-tert-Butyl phenyl)-1-(4-methoxy-phenyl)-prop-2-en-1-ol (Substanz 1a) und von 3-(4-tert-Butyl-phenyl)-1-(4-methoxy-phenyl)-propan-1-ol (Substanz 2a): Etwas Substanz wird in MTB-Ether gelöst und auf eine DC-Platte aufgetragen (DC Platte TLC Silica gel 60F254). Man chromatographiert mit Heptan/Essigsäureethylester 2/1. Nach anschließendem Besprühen mit frisch hergestellter Kaliumpermanganatlösung entfärbt sich die Sprühlösung an den Substanzflecken (4g NaOH in 96g H₂O, Zugabe von 1g KMnO₄). Die Rf-Werte liegen bei 0,61 (Substanz 2a) und 0,35 (Substanz 1a).

### Beispiel 5:

### Zubereitungen:

Im folgenden werden beispielhaft Rezepturen für kosmetische Zubereitungen angegeben. Entsprechende Zubereitungen können in gleicher Weise mit allen erfindungsgemäßen Verbindungen hergestellt werden.

Insbesondere können die in nachfolgenden Rezepturbeispielen verwendeten Substanzen (1a), (1b) und (1l) durch die entsprechenden Substanzen (2a), (2b) und (2l) vollständig oder teilweise ersetzt werden. Ferner können verwendete erfindungsgemäße Stoffe in isomerer Form enthalten sein. Dies beinhaltet Enantiomere wie auch Doppelbindungsisomere. Beispielsweise kann das E-Doppelbindungisomere ganz oder teilweise durch Z-Doppelbindungsisomere ersetzt sein.

Es sind die INCI-Bezeichnungen der handelsüblichen Verbindungen angegeben.

UV-Pearl, OMC steht für die Zubereitung mit der INCI-Bezeichnung: Water (for EU: Aqua), Ethylhexyl Methoxycinnamate, Silica, PVP, Chlorphenesin, BHT. Diese Zubereitung ist im Handel unter der Bezeichnung Eusolex®UV Pearl™OMC von der Merck KGaA, Darmstadt erhältlich.

Die anderen in den Tabellen angegebenen UV-Pearl sind jeweils analog zusammengesetzt, wobei OMC gegen die angegebenen UV-Filter ausgetauscht ist.

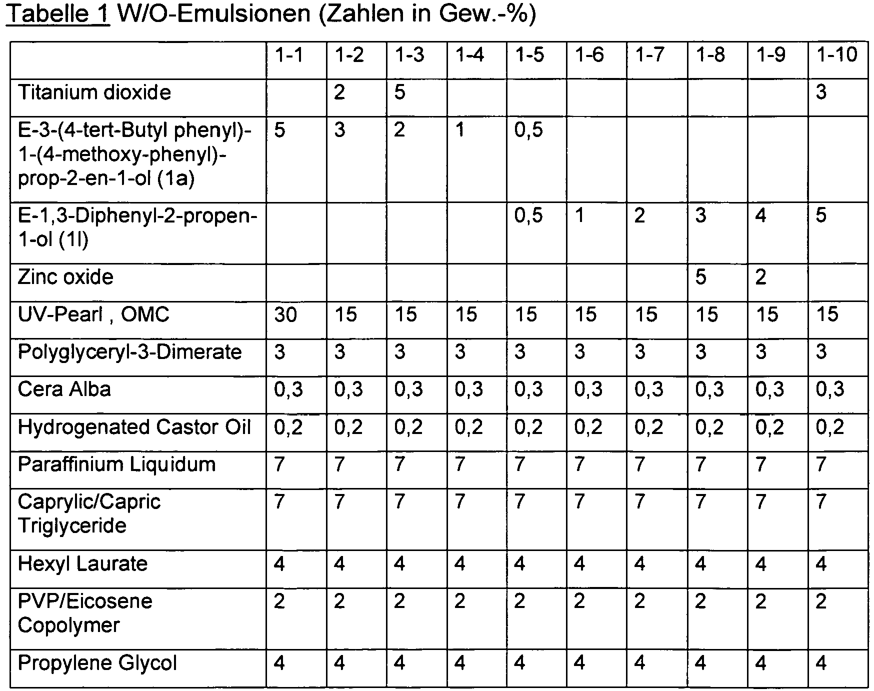

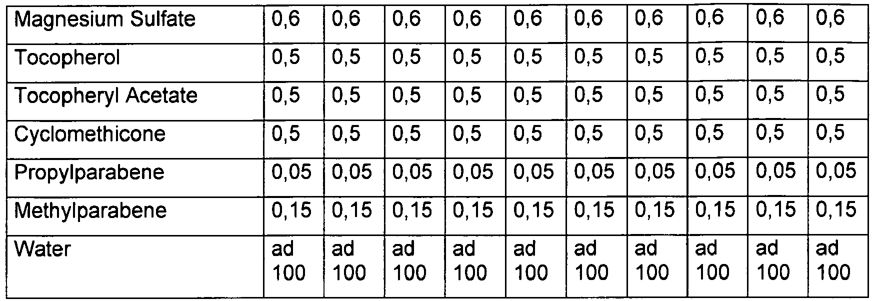

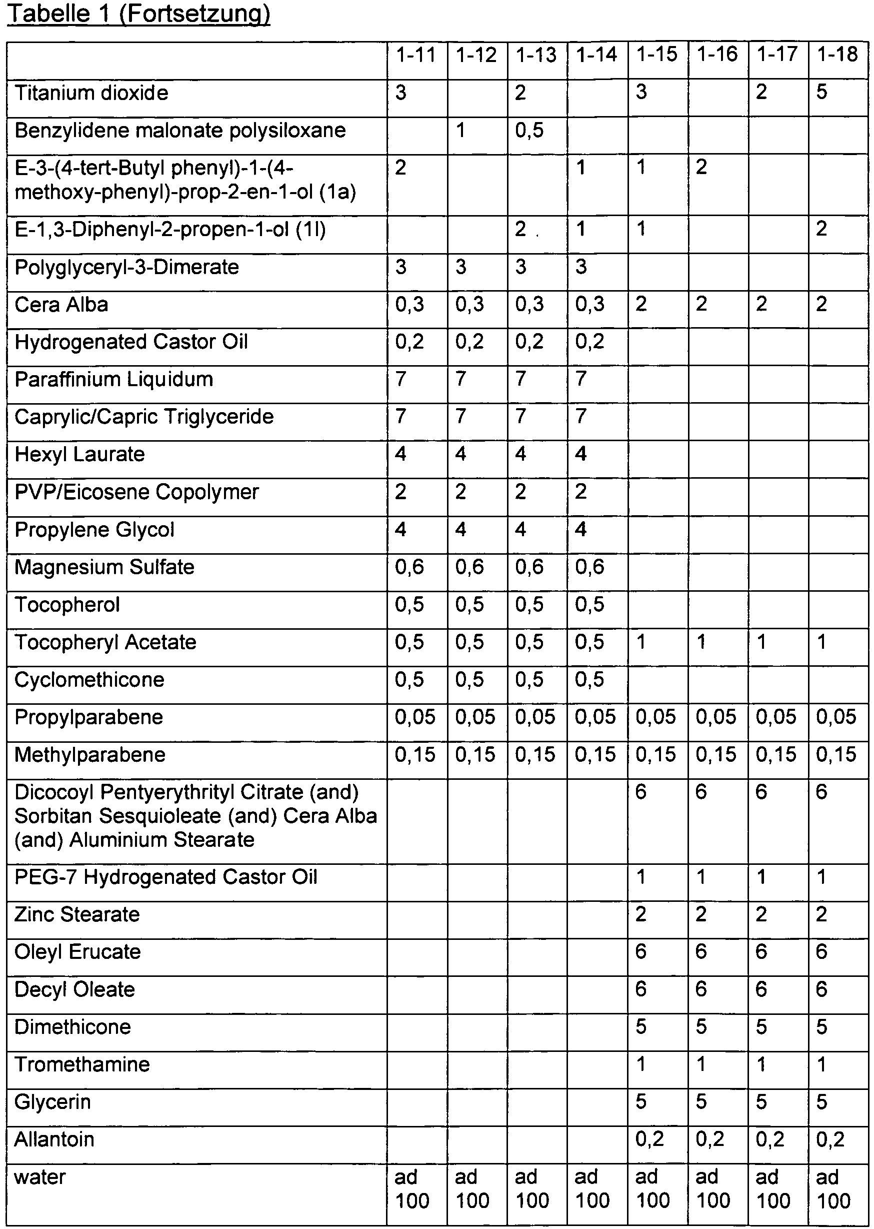

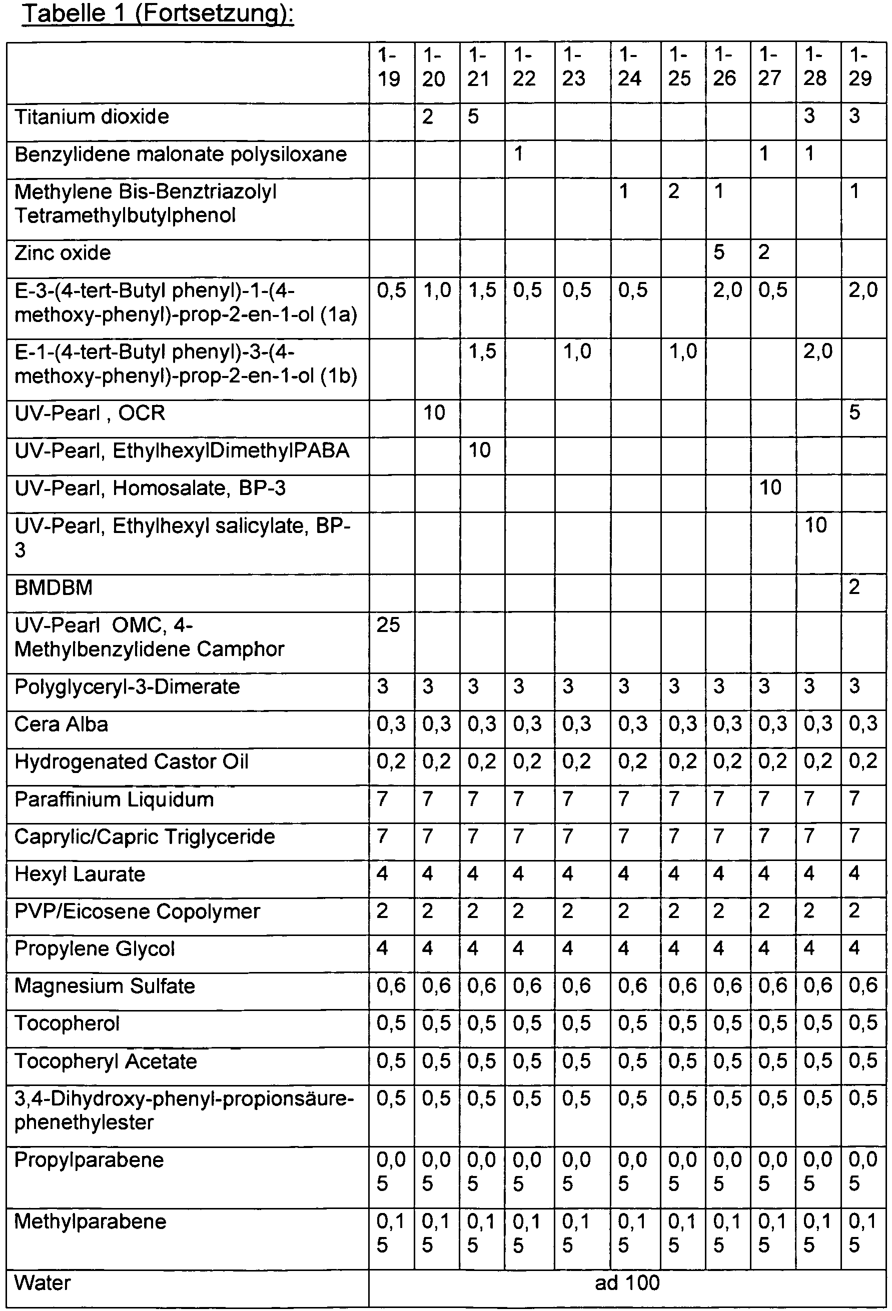

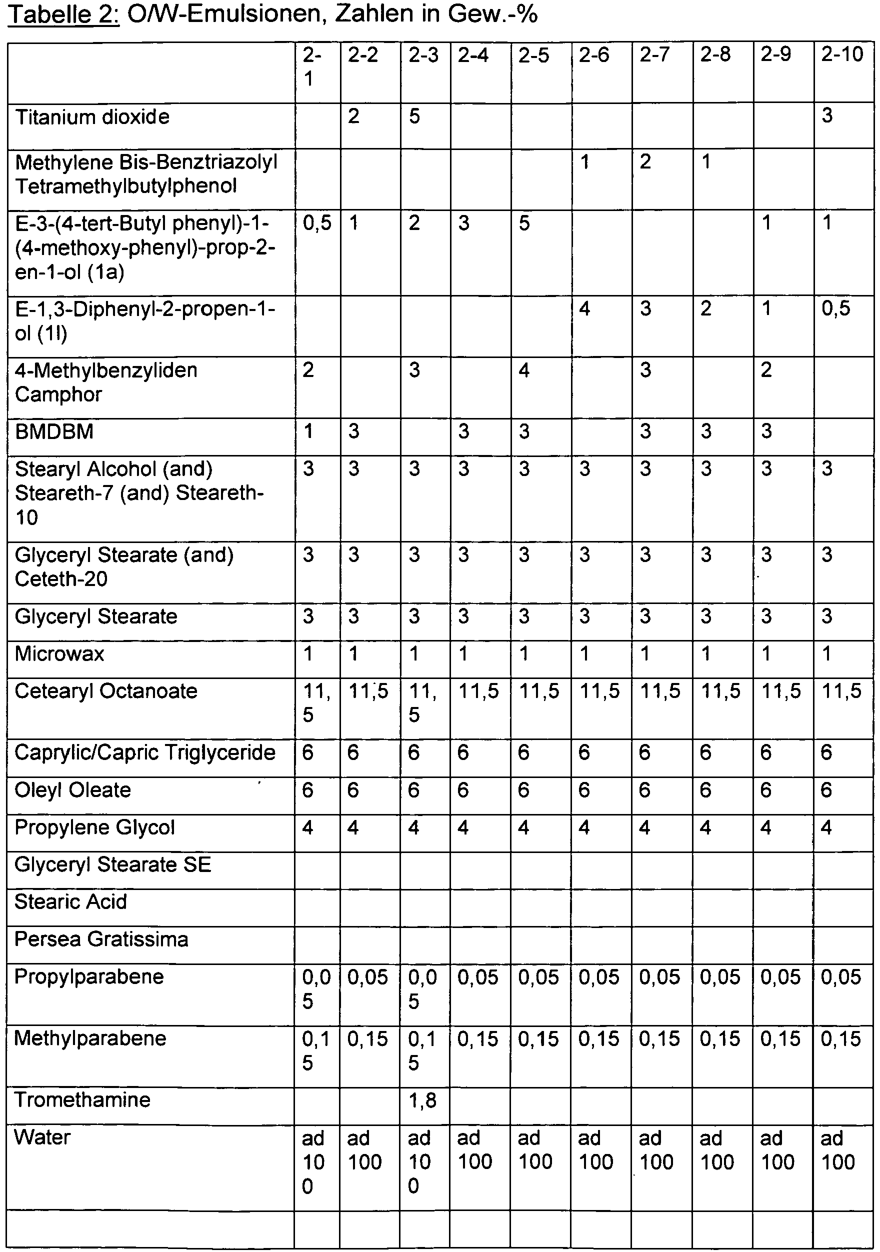

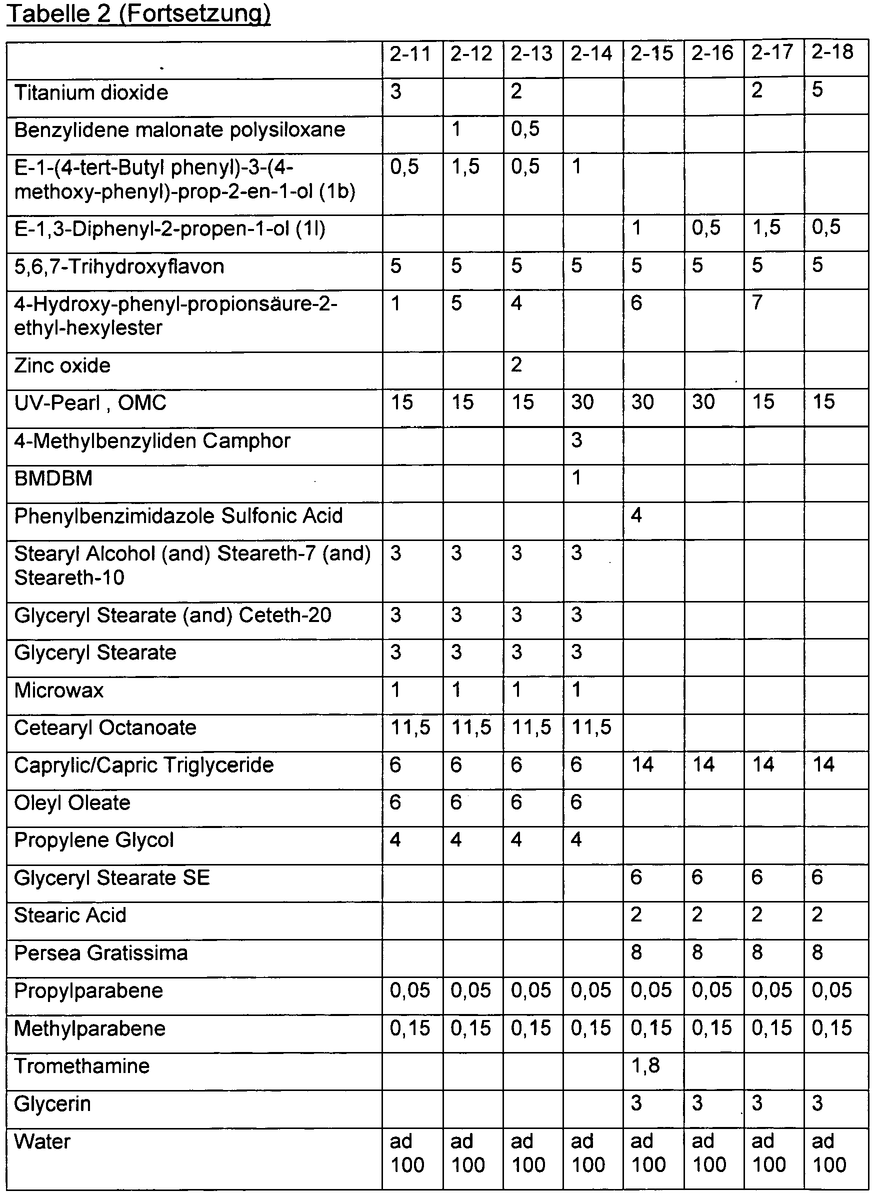

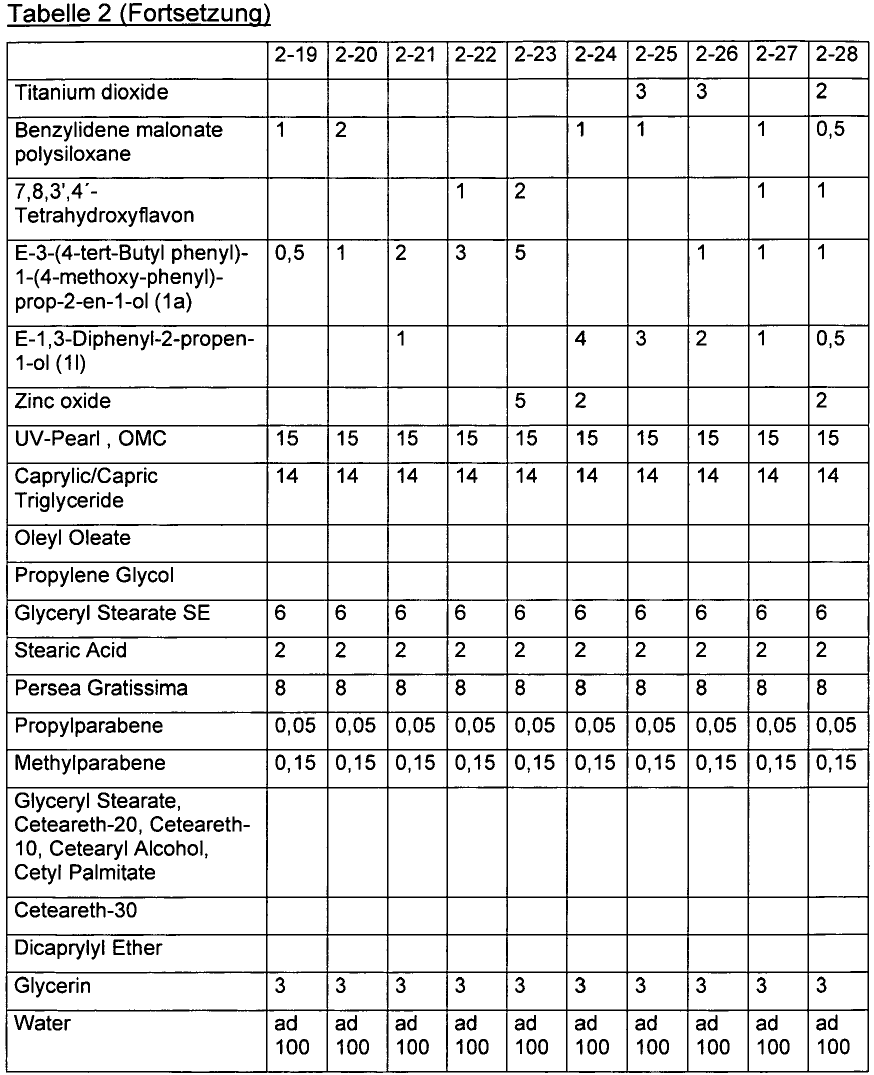

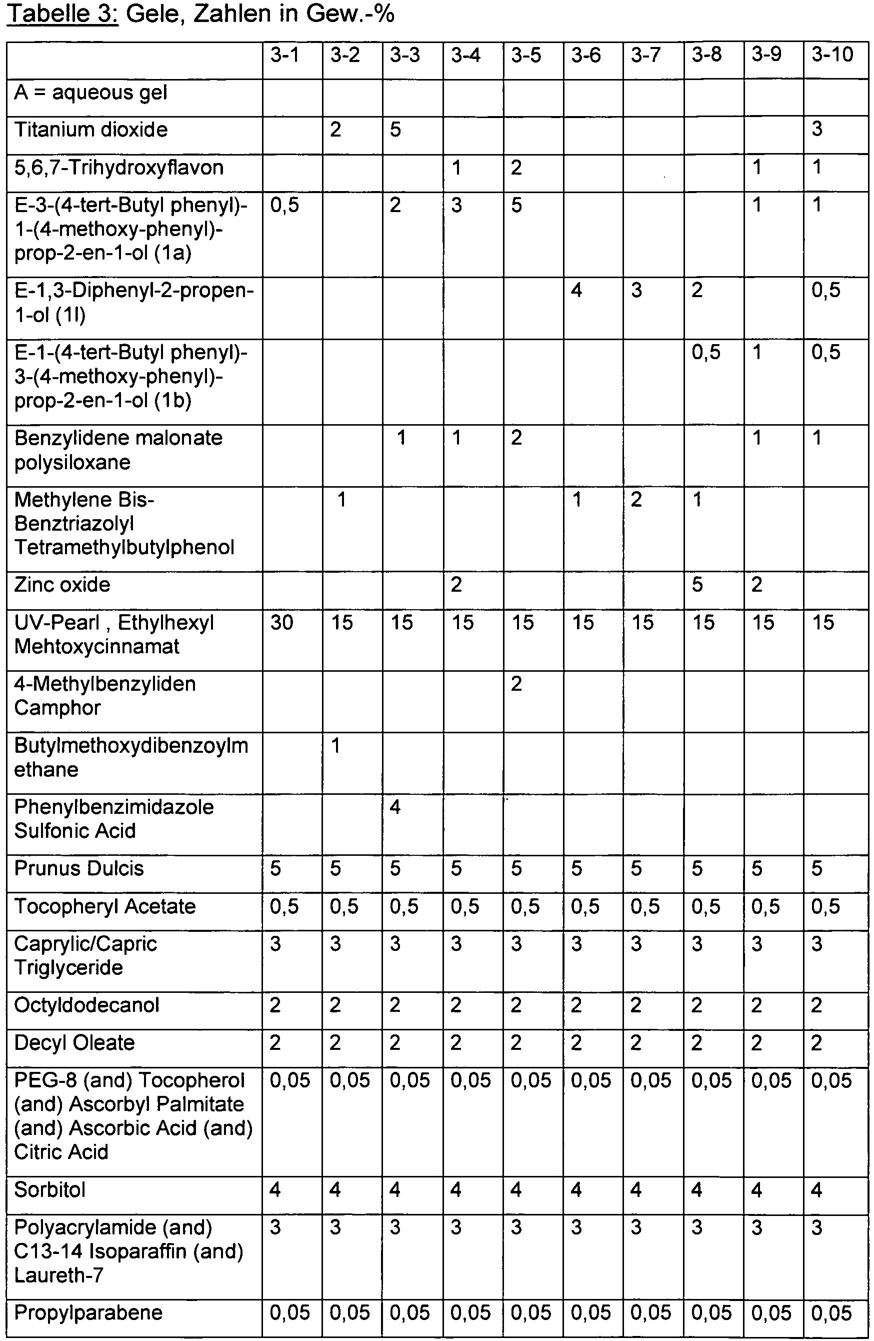

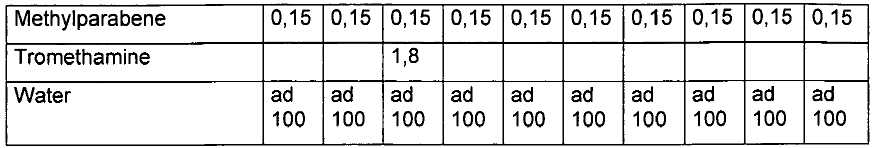

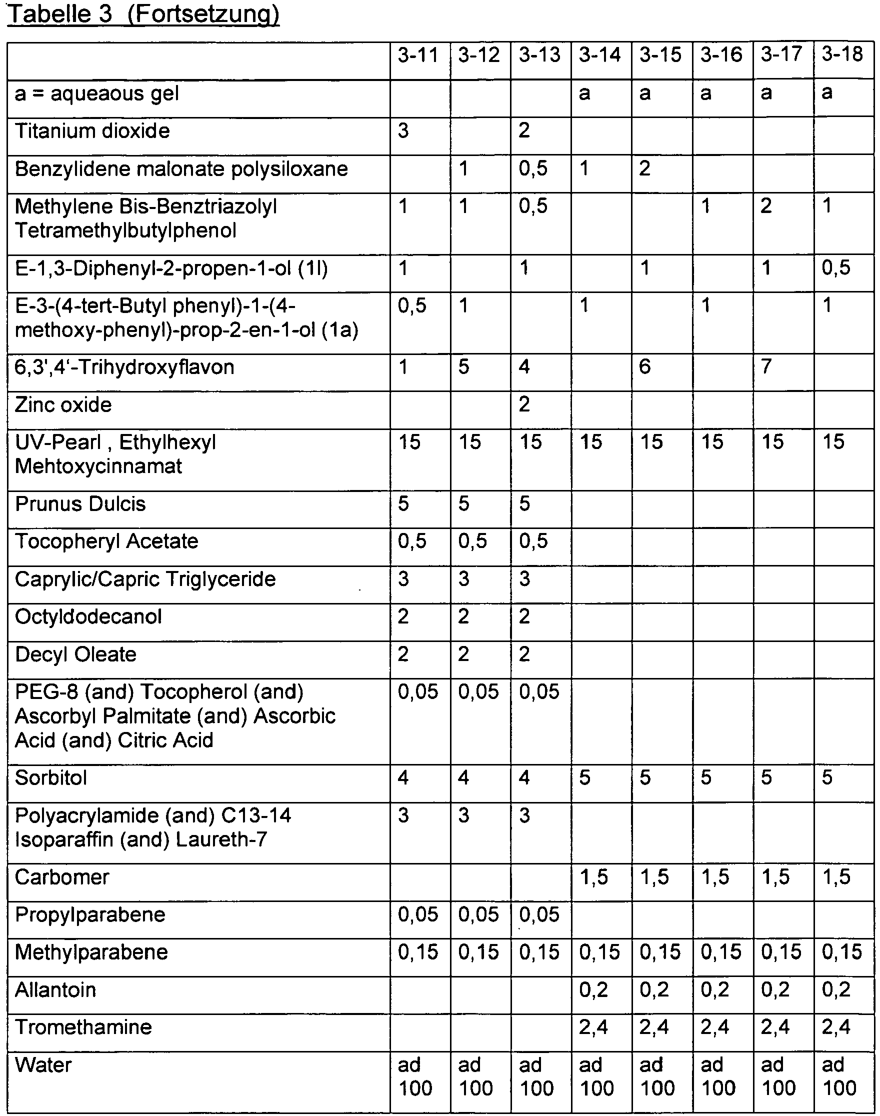

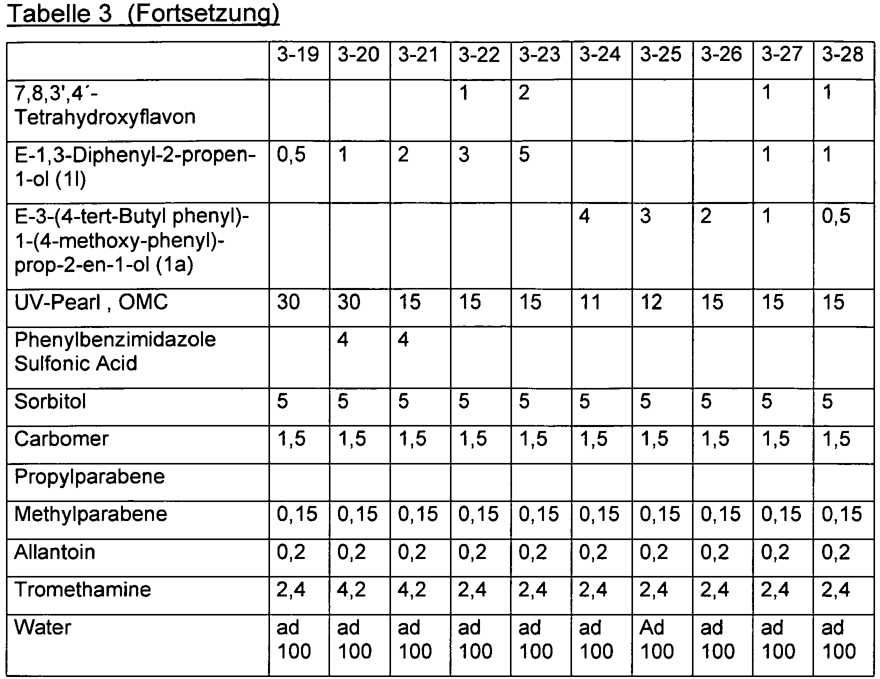

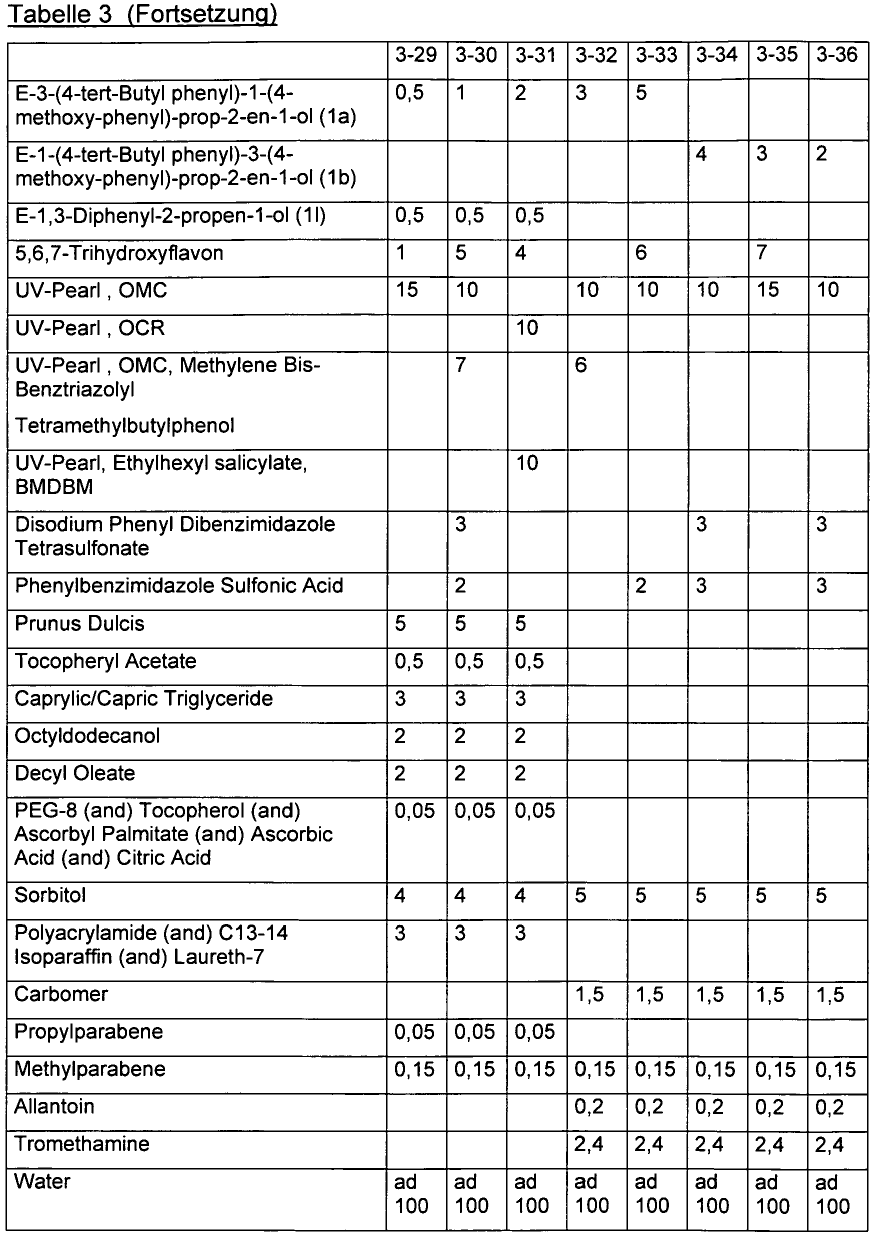

### Beispiel 6:

### Haarmascara

| Inhaltsstoffe | [%] |
|---|---|
| A | |
| PERLGLANZPIGMENT | 20,00 |

| B | |
|---|---|
| CETEARETH-25 | 1,80 |
| CETEARYLALCOHOL | 5,00 |
| DIMETHICONE | 1,00 |
| PHENOXYETHANOL, BUTYLPARABEN, ETHYLPARABEN, PROPYLPARABEN, METHYLPARABEN | 0,50 |

| C | |
|---|---|
| AQUA (WASSER) | ad 100 |
| POLYQUATERNIUM-16 | 3,0 |
| PROPYLENEGLYCOL | 1,80 |
| E-1,3-DIPHENYL-2-PROPEN-1-OL (1l) | 0,25 |
| E-3-(4-TERT-BUTYL PHENYL)-1-(4-METHOXYPHENYL)-PROP-2-EN-1-OL (1a) | 1,5 |

| D | |
|---|---|
| AQUA (WASSER) | 9,50 |
| HYDROXYPROPYLCELLULOSE | 0,50 |

| E | |
|---|---|
| AQUA (WASSER) | 9,50 |
| MAGNESIUM ALUMINIUM SILICATE | 0,50 |
| IMIDAZOLIDINYL UREA | 0,30 |

### Herstellverfahren:

Phase B auf 75 °C, Phase C auf 80 °C erhitzen. Phase B langsam unter Rühren zu Phase C zugeben. Unter Rühren auf 65 °C abkühlen und homogenisieren. Auf 40 °C abkühlen und Phasen D, E und F zu Phase B/C unter Rühren zugeben und wiederum homogenisieren. Nun das Perlglanzpigment unter Rühren zugeben. Auf Zimmertemperatur abkühlen und den pH-Wert auf 6,0-6,5 einstellen.

### Beispiel 7:

### Conditioner mit IR3535®

| Inhaltsstoffe | [%] |
|---|---|
| A | |
| ETHYLBUTYLACETYLAMINOPROPIONATE | 10,00 |
| PVP/VA COPOLYMER | 4,00 |
| PARFUM | 0,30 |
| QUATERNIUM-80 | 1,0 |
| PEG-40 HYDROGENATED CASTOR OIL | 1,00 |
| ALCOHOL | 15,00 |
| E-1,3-DIPHENYL-2-PROPEN-1-OL (1l)) | 0,5 |
| E-3-(4-TERT-BUTYL PHENYL)-1-(4-METHOXYPHENYL)-PROP-2-EN-1-OL (1a) | 0,5 |

| B | |
|---|---|
| CETRIMONIUM CHLORIDE | 0,50 |
| AQUA (WASSER) | Ad 100 |

| C | |
|---|---|
| COCAMIDOPROPYL BETAINE | 4,00 |

### Herstellverfahren:

Phasen A und B separat vermischen. Phase B zu Phase A unter Rühren hinzugeben. Phase C zugeben.

### Beispiel 8:

### Haarconditioner mit Perlglanzpigment

| Inhaltsstoffe | [%] |
|---|---|
| A | |
| PERLGLANZPIGMENT | 3,00 |
| DISODIUM EDTA | 0,05 |
| AQUA (WASSER) | ad 100 |

| B | |
|---|---|
| CETEARYLALCOHOL, BEHENTRIMONIUM METHOSULFATE | 5,00 |
| OCTYLDODECANOL | 1,10 |
| CETYLALCOHOL | 1,00 |
| GLYCERIN | 1,00 |
| BEHENTRIMONIUM CHLORIDE | 0,70 |
| METHOXY PEG/PPG-7/3 AMINOPROPYL DIMETHICONE | 0,70 |
| QUATERNIUM-80 | 1,0 |
| E-3-(4-TERT-BUTYL PHENYL)-1-(4-METHOXYPHENYL)-PROP-2-EN-1-OL (1a) | 0,1 |
| E-1,3-DIPHENYL-2-PROPEN-1-OL (1l) | 1,0 |
| VERBINDUNG NACH FORMEL IB-IAH | 2,0 |

| C | |
|---|---|
| COCODIMONIUM HYDROXYPROPYLSILIKAMINO ACIDS | 0,70 |
| PHENOXYETHANOL, BENZOIC ACID, DEHYDROACETIC ACID | 0,40 |
| CITRIC ACID | 0,20 |
| PARFUM | 0,60 |

### Herstellverfahren:

Das Perlglanzpigment und Titriplex III im Wasser der Phase A dispergieren. Die Bestandteile der Phasen A und B auf 75°C erhitzen. Phase B zu Phase A unter Rühren hinzugeben und homogenisieren. Auf 40°C abkühlen und die Bestandteile von Phase C zugeben. Auf 30°C abkühlen und wiederum ca. 30 sec. homogenisieren. Den pH-Wert auf 3,6-4,0 einstellen.

Anmerkungen: Empfohlene Perlglanzpigmente sind TIMIRON® Silberpigmente und TIMIRON® Interferenzpigmente der Fa. Merck.

## Patentansprüche

1. Nicht-therapeutische Verwendung einer oder mehrerer Verbindungen der Formel (1) und/ oder (2) 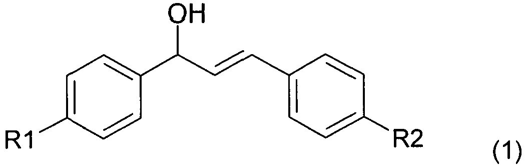 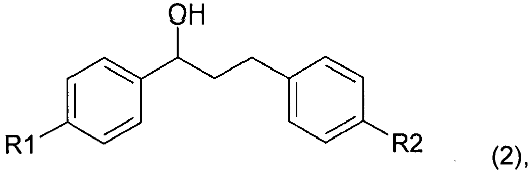 wobei R1 und R2 unabhängig voneinander für Wasserstoff, C1- bis C12-Alkoxy oder C1- bis C12-Alkyl stehen,
als Antioxidans.

2. Nicht-therapeutische Verwendung einer oder mehrerer Verbindungen der Formel (1) und/oder (2) gemäß Anspruch 1 zur Prophylaxe gegen oder zur Bekämpfung von oxidativem Stress.

3. Nicht-therapeutische Verwendung einer oder mehrerer Verbindungen der Formel (1) und/oder (2) gemäß Anspruch 1 zur Pflege, Konservierung oder Verbesserung des allgemeinen Zustands oder des Erscheinungsbildes der Haut oder Haare.

4. Nicht-therapeutische Verwendung einer oder mehrerer Verbindungen der Formel (1) und/oder (2) gemäß Anspruch 1 zur Prophylaxe gegen oder zur Bekämpfung von zeit- und/oder lichtinduzierten Alterungsprozessen der Haut oder Haare.

5. Verbindung der Formel (1) und/oder (2) 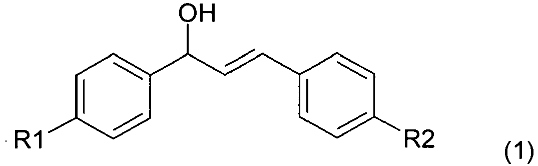 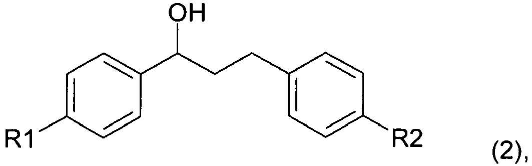 wobei R1 und R2 unabhängig voneinander für Wasserstoff, C1- bis C12-Alkoxy oder C1- bis C12-Alkyl stehen,
zur Prophylaxe gegen oder zur Bekämpfung von Sebumoxidation und/oder Akne.

6. Verwendung nach einem oder mehreren der Ansprüche 1 bis 4, **dadurch gekennzeichnet, dass** R1 für Wasserstoff, C1- bis C6-Alkyl oder C1- bis C6-Alkoxy steht.

7. Verwendung nach Anspruch 6, **dadurch gekennzeichnet, dass** R1 für Wasserstoff, Methoxy, Isopropyl oder t-Butyl steht.

8. Verwendung nach einem oder mehreren der Ansprüche 1 bis 4 und 6 bis 7, **dadurch gekennzeichnet, dass** R2 für Wasserstoff, C1- bis C6-Alkyl oder C1- bis C6-Alkoxy steht.

9. Verwendung nach Anspruch 8, **dadurch gekennzeichnet, dass** R2 für Wasserstoff, Methoxy, Isopropyl oder t-Butyl steht.

10. Verwendung nach einem oder mehreren der Ansprüche 1 bis 4 und 6 bis 10, **dadurch gekennzeichnet, dass** Formel (1) ausgewählt ist aus Verbindungen der Formeln (1a) bis (1p) und Formel (2) ausgewählt ist aus Verbindungen der Formeln (2a) bis (2p)
| | | | |
|---|---|---|---|
| 1a | 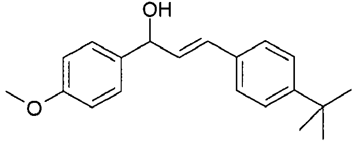 | 1b | 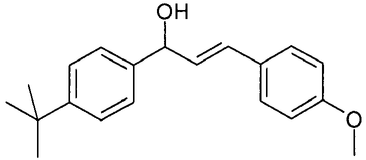 |
| 1c | 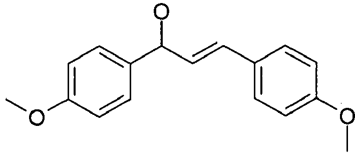 | 1d | 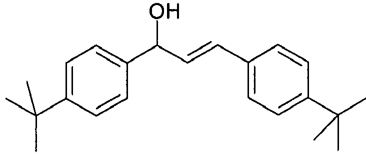 |
| 1e | 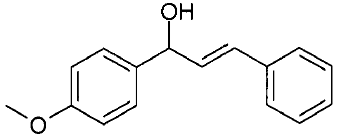 | 1f | 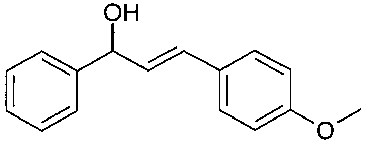 |
| 1g | 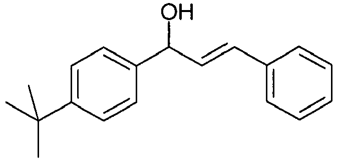 | 1h | 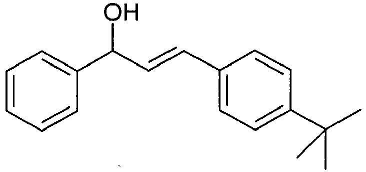 |
| 1i | 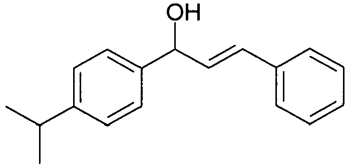 | 1j | 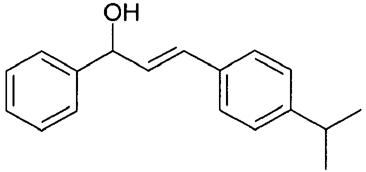 |
| 1k | 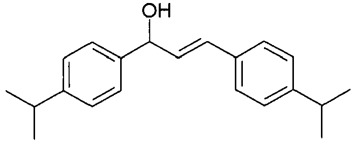 | 1l | 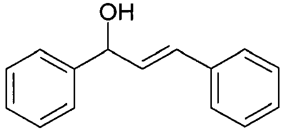 |
| 1m | 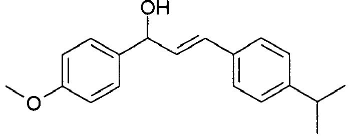 | 1n | 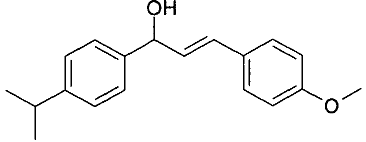 |
| 1o | 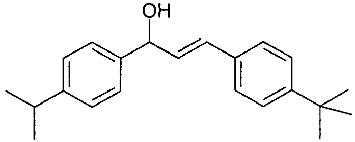 | 1p | 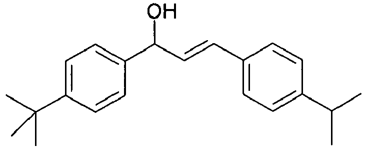 |
| | | | |
|---|---|---|---|
| 2a | 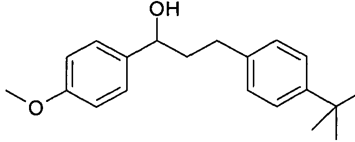 | 2b | 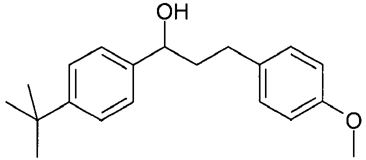 |
| 2c | 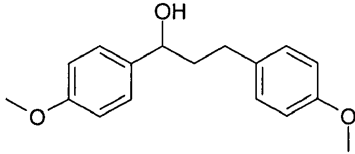 | 2d | 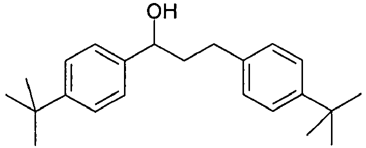 |
| 2e | 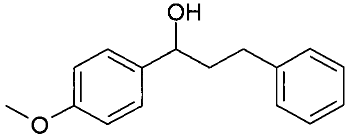 | 2f | 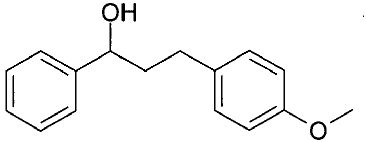 |
| 2g | 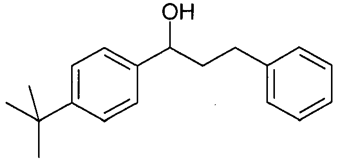 | 2h | 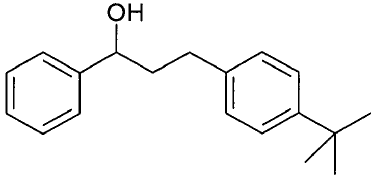 |
| 2i | 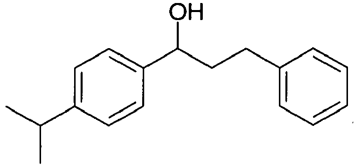 | 2j | 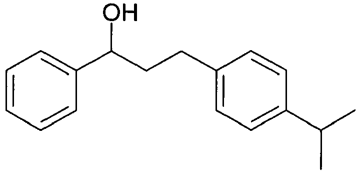 |
| 2k | 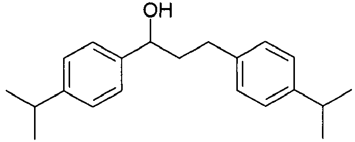 | 2l | 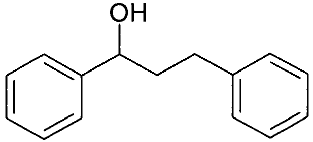 |
| 2m | 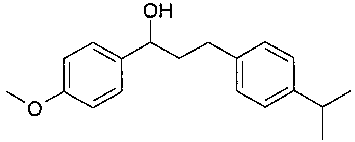 | 2n | 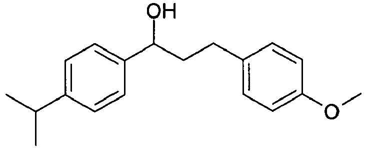 |
| 2o | 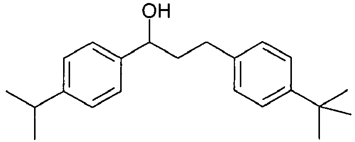 | 2p | 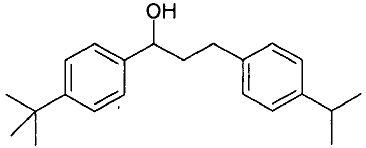 |

11. Zubereitung enthaltend mindestens eine Verbindung der Formel (1) und/ oder (2) 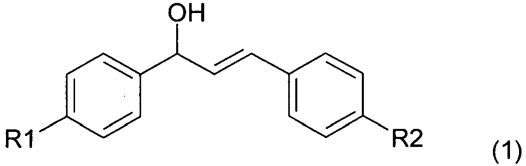 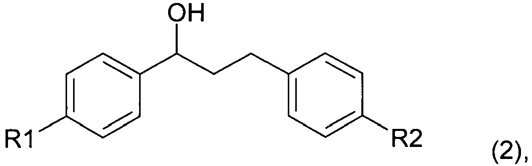 wobei R1 und R2 unabhängig voneinander für Wasserstoff, C1- bis C12-Alkoxy oder C1- bis C12-Alkyl stehen,
und mindestens einen für topische Anwendungen geeigneten Träger.

12. Zubereitung nach Anspruch 11, **dadurch gekennzeichnet, dass** die mindestens eine Verbindung der Formel (1) oder (2) in einer Menge von 0,01 bis 20 Gew.-% enthalten ist.

13. Verfahren zur Herstellung einer Zubereitung nach Anspruch 11 oder 12, **dadurch gekennzeichnet, dass** die mindestens eine Verbindung der Formel (1) oder (2) mit dem für topische Anwendungen geeigneten Träger vermischt wird.

14. Verbindung der Formel (1) oder (2) 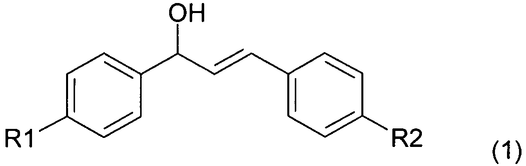 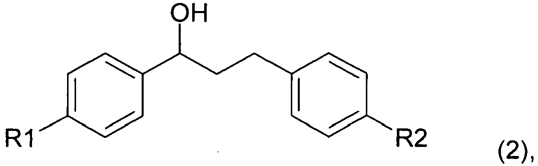 wobei R1 und R2 unabhängig voneinander für Wasserstoff, C1- bis C12-Alkoxy oder C1- bis C12-Alkyl stehen,
wobei Verbindungen der Formel (1) ausgeschlossen sind, in denen
R1=R2=H (11),
R1 =R2=Methyl,
R1=R2=Ethyl,
R1=R2=i-Butyl,
R1=R2=Methoxy (1c),
R1=Methyl, R2=H,
R1=H, R2=Methyl,
R1=Ethyl, R2=H,
R1=n-Heptyl, R2=H,
R1=Methoxy, R2=H (1e),
R1=H, R2=Methoxy (1f),
R1=Methyl, R2=Methoxy oder
R1=Methoxy, R2=Methyl,
und wobei Verbindungen der Formel (2) ausgewählt sind aus dem Verbindungen
| | | | |
|---|---|---|---|
| 2a | 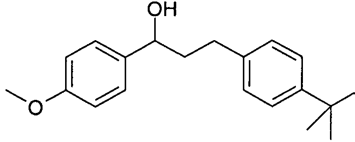 | 2b | 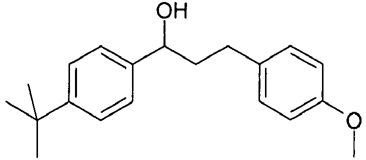 |
| | | 2d | 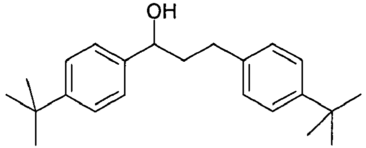 |
| 2k | 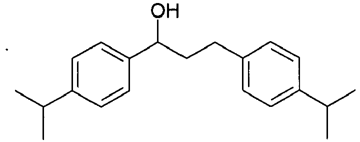 | | |
| 2m | 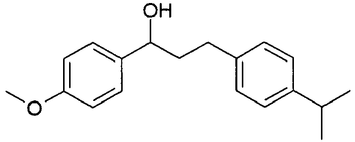 | | |
| 2o | 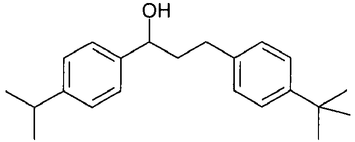 | 2p | 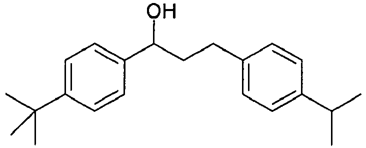 |

15. Verfahren zur Herstellung einer Verbindung der Formel (1) oder (2) nach Anspruch 14, **dadurch gekennzeichnet, dass** eine Verbindung der Formel (1-A) oder (2-A) 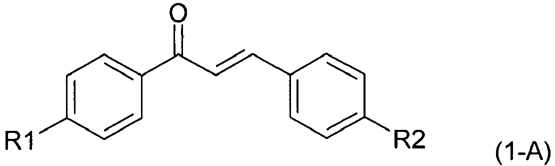 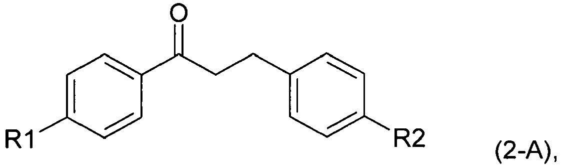 wobei die Reste R1 und R2 wie für Formel (1) bzw. Formel (2) definiert sind,
durch katalytische Hydrierung zu einer Verbindung der Formel (1) oder (2) nach Anspruch 14 umgesetzt wird.

## Claims

1. Non-therapeutic use of one or more compounds of the formula (1) and/or (2) 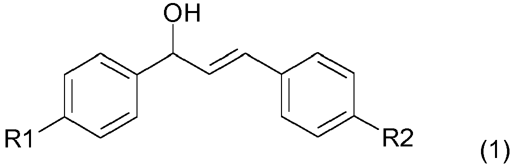 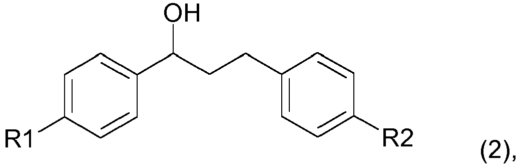 where R1 and R2 stand, independently of one another, for hydrogen, C1- to C12-alkoxy or C1- to C12-alkyl,
as antioxidant.

2. Non-therapeutic use of one or more compounds of the formula (1) and/or (2) according to Claim 1 for prophylaxis against or for combating of oxidative stress.

3. Non-therapeutic use of one or more compounds of the formula (1) and/or (2) according to Claim 1 for the care, preservation or improvement of the general condition or appearance of the skin or hair.

4. Non-therapeutic use of one or more compounds of the formula (1) and/or (2) according to Claim 1 for prophylaxis against or for combating of time- and/or light-induced ageing processes of the skin or hair.

5. Compound of the formula (1) and/ or (2) 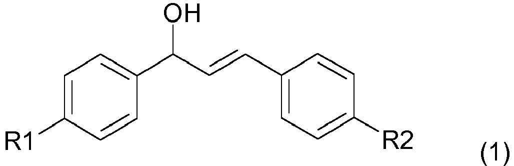 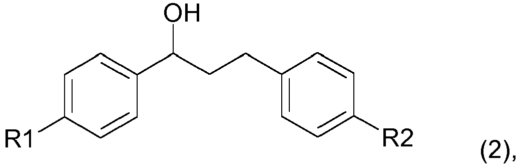 where R1 and R2 stand, independently of one another, for hydrogen, C1- to C12-alkoxy or C1- to C12-alkyl,
for prophylaxis against or for combating of sebum oxidation and/or acne.

6. Use according to one or more of Claims 1 to 4, **characterised in that** R1 stands for hydrogen, C1- to C6-alkyl or C1- to C6-alkoxy.

7. Use according to Claim 6, **characterised in that** R1 stands for hydrogen, methoxy, isopropyl or t-butyl.

8. Use according to one or more of Claims 1 to 4 and 6 to 7, **characterised in that** R2 stands for hydrogen, C1- to C6-alkyl or C1- to C6-alkoxy.

9. Use according to Claim 8, **characterised in that** R2 stands for hydrogen, methoxy, isopropyl or t-butyl.

10. Use according to one or more of Claims 1 to 4 and 6 to 10, **characterised in that** formula (1) is selected from compounds of the formulae (1a) to (1p) and formula (2) is selected from compounds of the formulae (2a) to (2p)
| | | | |
|---|---|---|---|
| 1a | 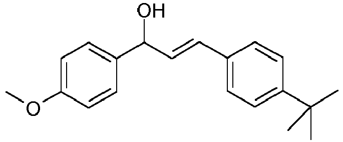 | 1b | 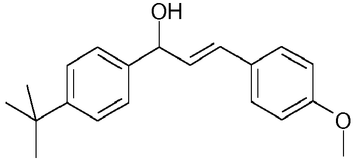 |
| 1c | 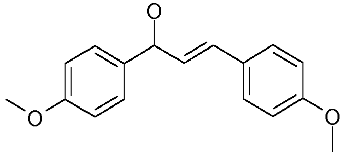 | 1d | 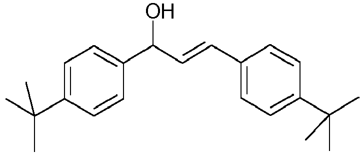 |
| 1e | 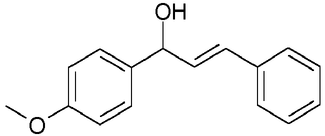 | 1f | 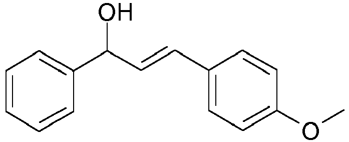 |
| 1g | 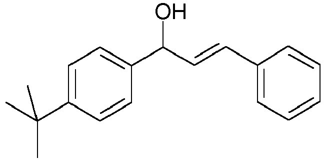 | 1h | 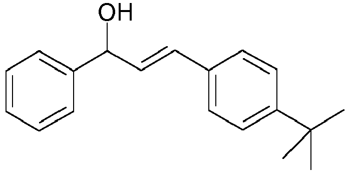 |
| 1i | 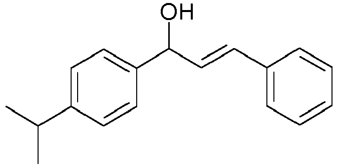 | 1j | 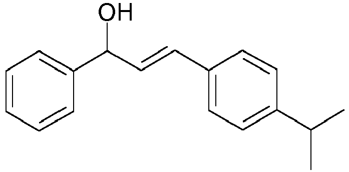 |
| 1k | 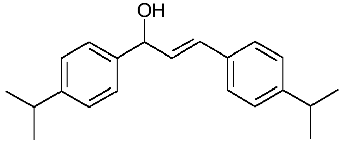 | 1l | 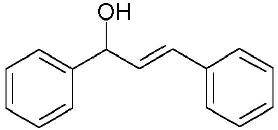 |
| 1m | 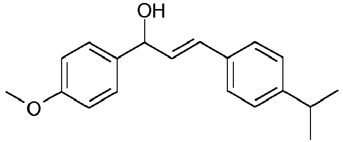 | 1n | 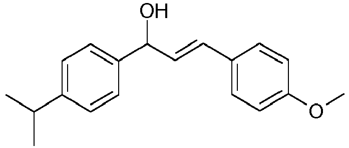 |
| 1o | 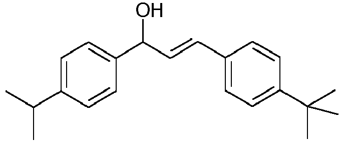 | 1p | 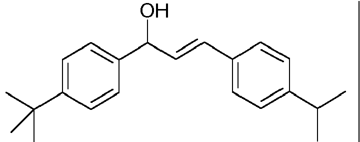 |
| | | | |
|---|---|---|---|
| 2a | 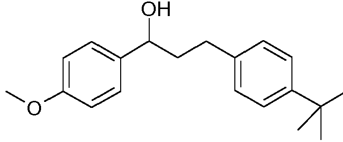 | 2b | 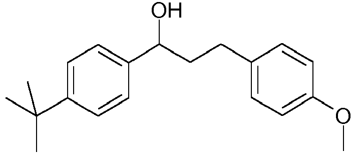 |
| 2c | 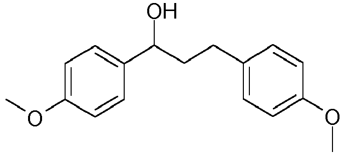 | 2d | 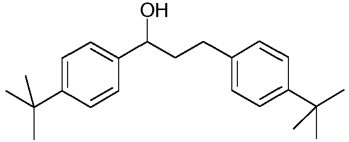 |
| 2e | 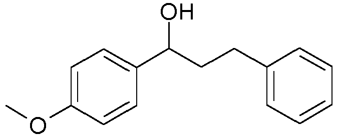 | 2f | 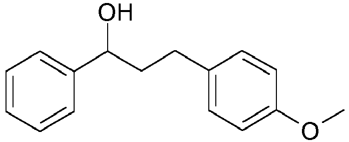 |
| 2g | 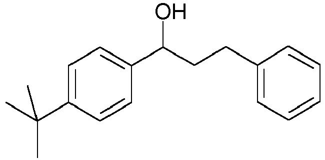 | 2h | 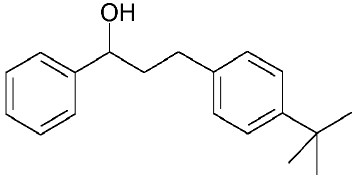 |
| 2i | 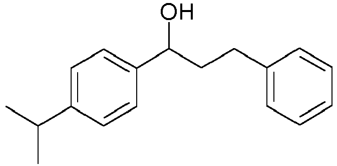 | 2j | 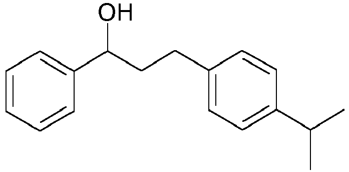 |
| 2k | 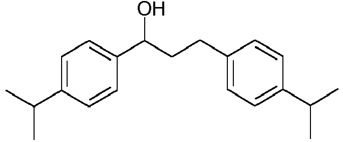 | 2l | 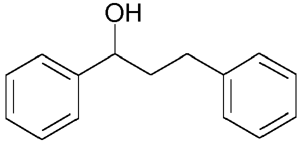 |
| 2m | 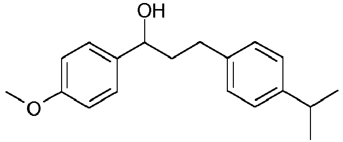 | 2n | 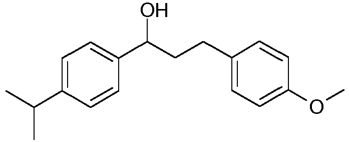 |
| 2o | 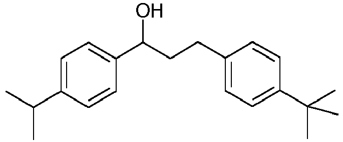 | 2p | 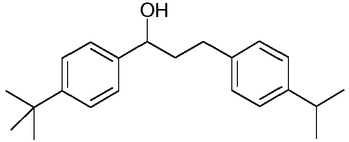 |

11. Preparation comprising at least one compound of the formula (1) and/ or (2) 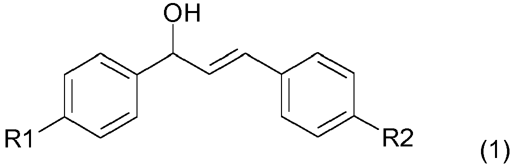 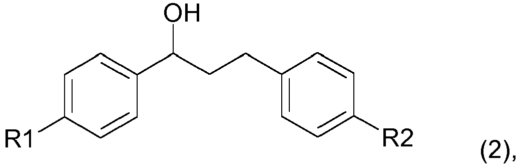 where R1 and R2 stand, independently of one another, for hydrogen, C1- to C12-alkoxy or C1- to C12-alkyl,
and at least one vehicle which is suitable for topical applications.

12. Preparation according to Claim 11, **characterised in that** the at least one compound of the formula (1) or (2) is present in an amount of 0.01 to 20% by weight.

13. Process for the preparation of a preparation according to Claim 11 or 12, **characterised in that** the at least one compound of the formula (1) or (2) is mixed with the vehicle which is suitable for topical applications.

14. Compound of the formula (1) or (2) 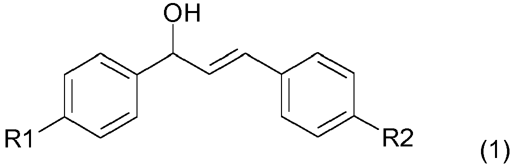 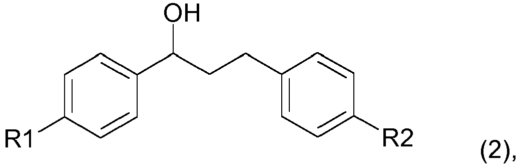 where R1 and R2 stand, independently of one another, for hydrogen, C1- to C12-alkoxy or C1- to C12-alkyl,
where compounds of the formula (1) in which
R1=R2=H (11),
R1=R2=methyl,
R1=R2=ethyl,
R1=R2=i-butyl,
R1=R2=methoxy (1c),
R1=methyl, R2=H,
R1=H, R2=methyl,
R1=ethyl, R2=H,
R1=n-heptyl, R2=H,
R1=methoxy, R2=H (1e),
R1=H, R2=methoxy (1f),
R1=methyl, R2=methoxy or
R1=methoxy, R2=methyl,
are excluded
and where compounds of the formula (2) are selected from the compounds
| | | | |
|---|---|---|---|
| 2a | 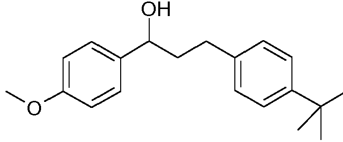 | 2b | 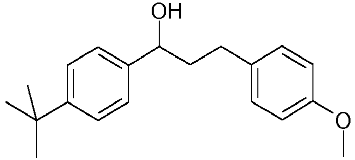 |
| | | 2d | 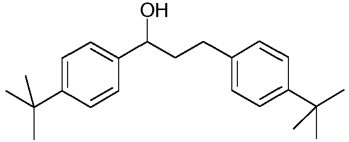 |
| 2k | 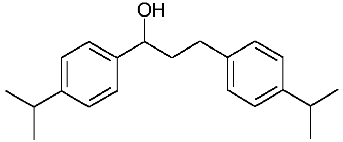 | | |
| 2m | 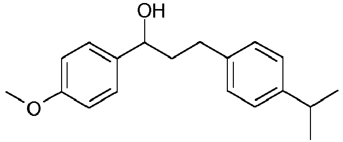 | | |
| 2o | 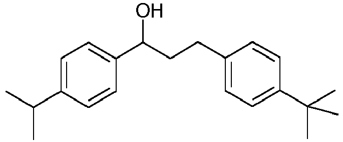 | 2p | 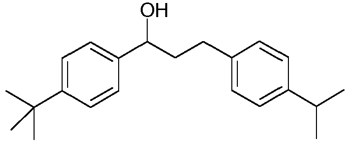 |

15. Process for the preparation of a compound of the formula (1) or (2) according to Claim 14, **characterised in that** a compound of the formula (1-A) or (2-A) 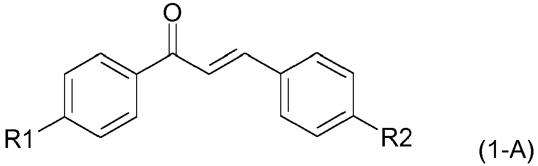 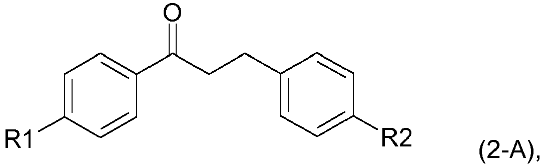 where the radicals R1 and R2 are defined as for formula (1) or formula (2),
is converted into a compound of the formula (1) or (2) according to Claim 14 by catalytic hydrogenation.

## Revendications

1. Utilisation non thérapeutique d'un ou plusieurs composés de formule (1) et/ou (2) 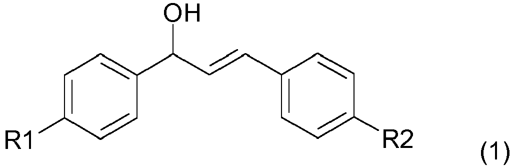 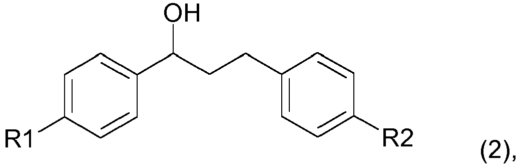 où R1 et R2 représentent, indépendamment l'un de l'autre, hydrogène, C1- à C12-alcoxy ou C1- à C12-alkyle,
comme antioxydant.

2. Utilisation non thérapeutique d'un ou plusieurs composés de formule (1) et/ou (2) selon la revendication 1, pour une prophylaxie ou pour lutter contre le stress oxydatif.

3. Utilisation non thérapeutique d'un ou plusieurs composés de formule (1) et/ou (2) selon la revendication 1, pour le soin, le maintien ou l'amélioration de l'état général ou de l'aspect de la peau ou des cheveux.

4. Utilisation non thérapeutique d'un ou plusieurs composés de formule (1) et/ou (2) selon la revendication 1, pour une prophylaxie ou pour lutter contre les processus de vieillissement de la peau ou des cheveux induits par le temps et/ou la lumière.

5. Composé de formule (1) et/ou (2) 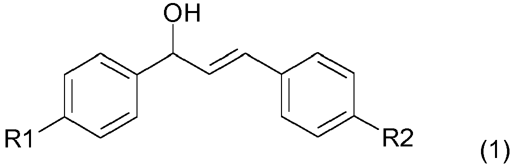 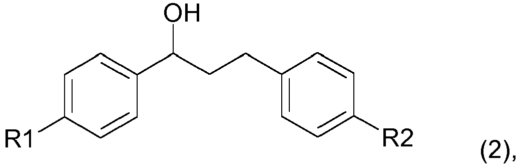 où R1 et R2 représentent, indépendamment l'un de l'autre, hydrogène, C1- à C12-alcoxy ou C1- à C12-alkyle,
pour une prophylaxie ou pour lutter contre l'oxydation du sébum et/ou l'acné.

6. Utilisation selon l'une ou plusieurs parmi les revendications 1 à 4, **caractérisée en ce que** R1 représente hydrogène, C1- à C6-alkyle ou C1- à C6-alcoxy.

7. Utilisation selon la revendication 6, **caractérisée en ce que** R1 représente hydrogène, méthoxy, isopropyle ou t-butyle.

8. Utilisation selon l'une ou plusieurs parmi les revendications 1 à 4 et 6 à 7, **caractérisée en ce que** R2 représente hydrogène, C1- à C6-alkyle ou C1- à C6-alcoxy.

9. Utilisation selon la revendication 8, **caractérisée en ce que** R2 représente hydrogène, méthoxy, isopropyle ou t-butyle.

10. Utilisation selon l'une ou plusieurs parmi les revendications 1 à 4 et 6 à 10, **caractérisée en ce que** la formule (1) est choisie parmi les composés de formules (1a) à (1p) et la formule (2) est choisie parmi les composés de formules (2a) à (2p)
| | | | |
|---|---|---|---|
| 1a | 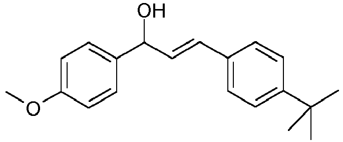 | 1b | 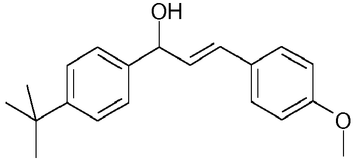 |
| 1c | 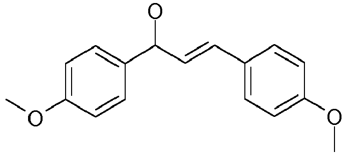 | 1d | 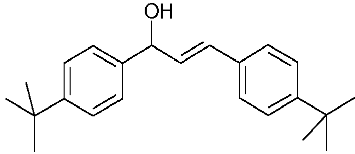 |
| 1e | 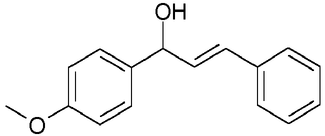 | 1f | 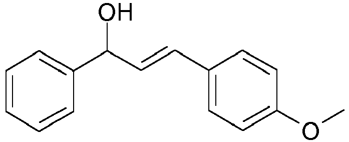 |
| 1g | 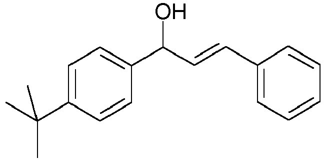 | 1h | 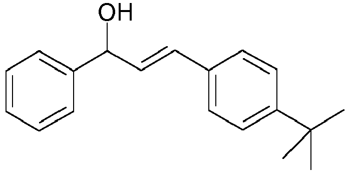 |
| 1i | 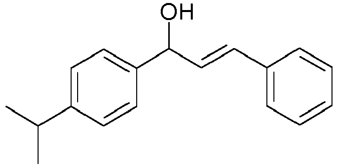 | 1j | 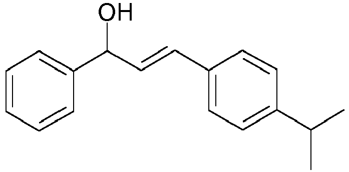 |
| 1k | 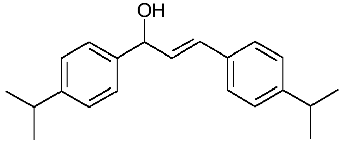 | 1l | 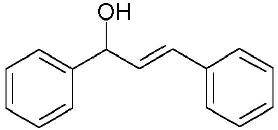 |
| 1m | 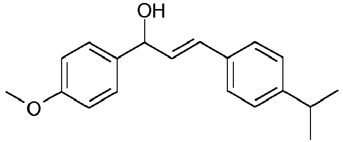 | 1n | 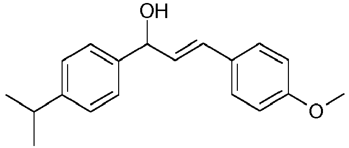 |
| 1o | 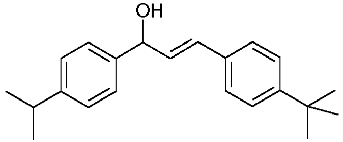 | 1p | 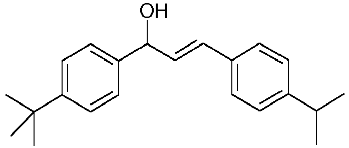 |
| | | | |
|---|---|---|---|
| 2a | 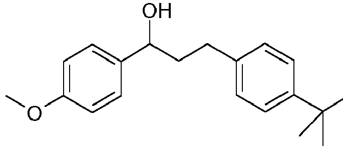 | 2b | 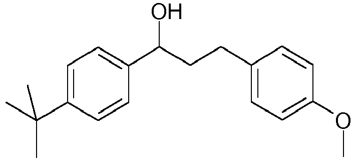 |
| 2c | 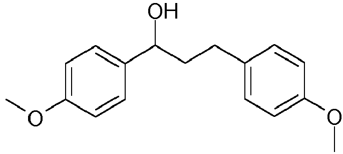 | 2d | 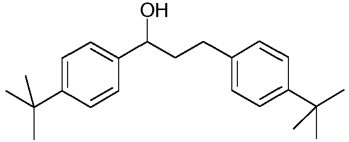 |
| 2e | 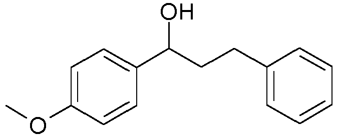 | 2f | 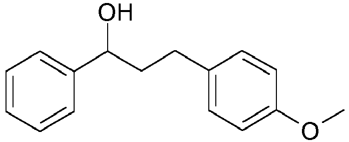 |
| 2g | 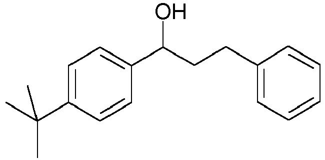 | 2h | 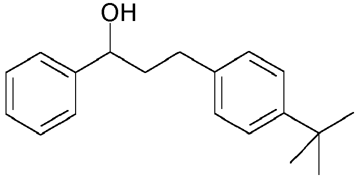 |
| 2i | 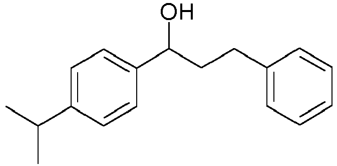 | 2j | 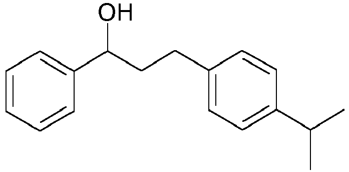 |
| 2k | 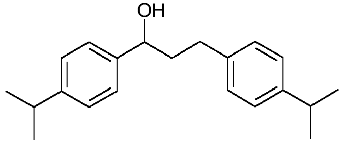 | 2l | 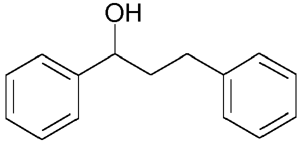 |
| 2m | 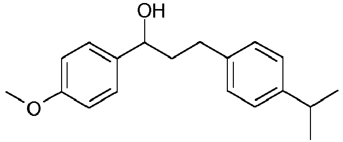 | 2n | 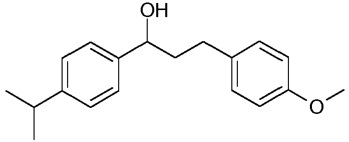 |
| 2o | 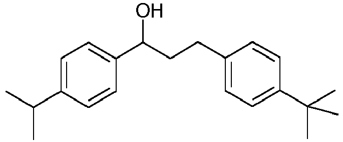 | 2p | 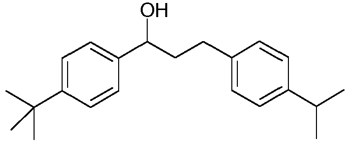 |

11. Préparation comprenant au moins un composé de formule (1) et/ou (2) 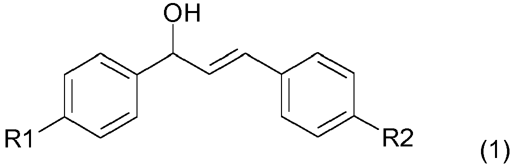 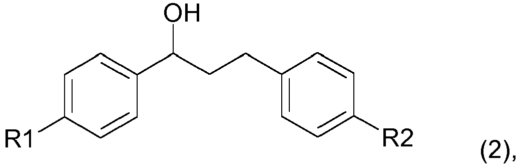 où R1 et R2 représentent, indépendamment l'un de l'autre, hydrogène, C1- à C12-alcoxy ou C1- à C12-alkyle,
et au moins un véhicule qui est convenable pour des applications topiques.

12. Préparation selon la revendication 11, **caractérisée en ce que** le au moins un composé de formule (1) ou (2) est présent selon une quantité allant de 0,01 à 20% en poids.

13. Procédé de préparation d'une préparation selon la revendication 11 ou 12, **caractérisé en ce que** le au moins un composé de formule (1) ou (2) est mélangé avec le véhicule qui est convenable pour des applications topiques.

14. Composé de formule (1) ou (2) 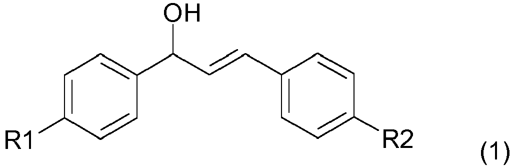 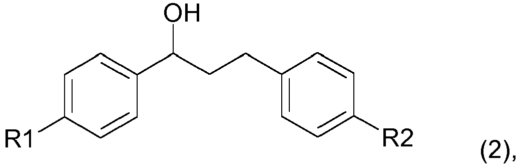 où R1 et R2 représentent, indépendamment l'un de l'autre, hydrogène, C1- à C12-alcoxy ou C1- à C12-alkyle,
où les composés de formule (1) dans lesquels
R1=R2=H (11),
R1=R2=méthyle,
R1=R2=éthyle,
R1=R2=i-butyle,
R1=R2=méthoxy (1c),
R1=méthyle, R2=H,
R1=H, R2=méthyle,
R1=éthyle, R2=H,
R1=n-heptyle, R2=H,
R1=méthoxy, R2=H (1e),
R1=H, R2=méthoxy (1f),
R1=méthyle, R2=méthoxy ou
R1=méthoxy, R2=méthyle,
sont exclus
et où les composés de formule (2) sont choisis parmi les composés
| | | | |
|---|---|---|---|
| 2a | 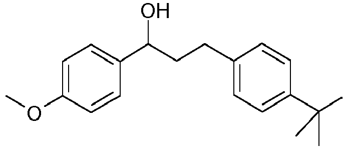 | 2b | 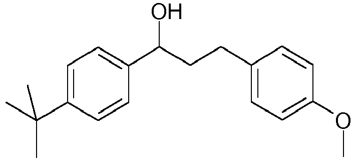 |
| | | 2d | 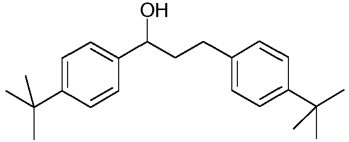 |
| 2k | 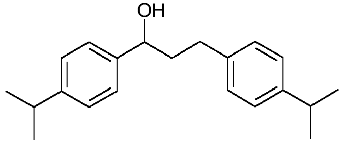 | | |
| 2m | 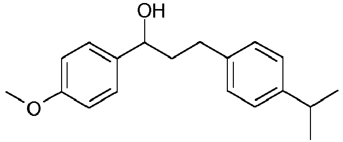 | | |
| 2o | 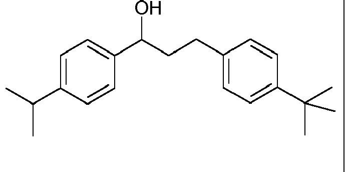 | 2p | 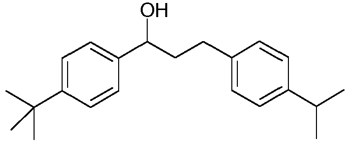 |

15. Procédé de préparation d'un composé de formule (1) ou (2) selon la revendication 14, **caractérisé en ce qu'**un composé de formule (1-A) ou (2-A) 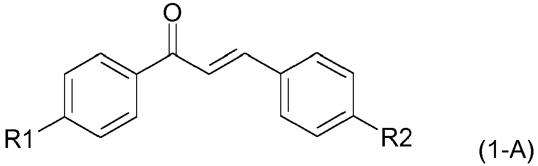 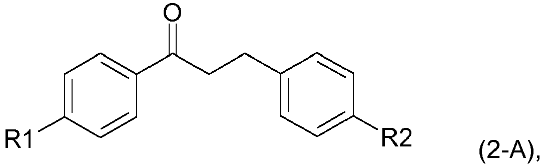 où les radicaux R1 et R2 sont tels que définis pour la formule (1) ou la formule (2),
est converti en un composé de formule (1) ou (2) selon la revendication 14 par hydrogénation catalytique.
